(19) Europäisches Patentamt
European Patent Office
Office européen des brevets

(11) Veröffentlichungsnummer: **0 010 715**
**B1**

# (12) EUROPÄISCHE PATENTSCHRIFT

(45) Veröffentlichungstag der Patentschrift: **27.01.82**

(51) Int. Cl.[3]: **C 07 C 131/00**, A 01 N 37/22

(21) Anmeldenummer: **79104081.9**

(22) Anmeldetag: **22.10.79**

(54) **Neue, eine Oximgruppe enthaltende N-Alkylhalogenacetanilide, Verfahren zu ihrer Herstellung, sie enthaltende herbizide Mittel, ein Verfahren zur Bekämpfung von Unkräutern und ein Verfahren zur Herstellung von herbiziden Mitteln.**

(30) Priorität: **03.11.78 DE 2847827**
**31.07.79 DE 2931103**

(43) Veröffentlichungstag der Anmeldung: **14.05.80 Patentblatt 80/10**

(45) Bekanntmachung des Hinweises auf die Patenterteilung: **27.01.82 Patentblatt 82/4**

(84) Benannte Vertragsstaaten: **AT BE CH DE FR GB IT NL SE**

(56) Entgegenhaltungen:
**AT-B 341 500**
**DE-A1-2 726 253**

(73) Patentinhaber: **BAYER AG, Zentralbereich Patente, Marken und Lizenzen, D-5090 Leverkusen 1, Bayerwerk (DE)**

(72) Erfinder: **Stetter, Jörg, Dr., Pahlkestrasse 3, D-5600 Wuppertal 1 (DE)**
Erfinder: **Draber, Wilfried, Dr., In den Birken 81, D-5600 Wuppertal 1 (DE)**
Erfinder: **Regel, Erik, Bergerheide 72a, D-5600 Wuppertal 1 (DE)**
Erfinder: **Eue, Ludwig, Dr., Paul-Klee-Strasse 36, D-5090 Leverkusen (DE)**
Erfinder: **Schmidt, Robert Rudolf, Dr., Hahnenweg 5, D-5000 Köln 80 (DE)**

EP 0 010 715 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Neue, eine Oximgruppe enthaltende N-Alkylhalogenacetanilide, Verfahren zu ihrer Herstellung, sie enthaltende herbizide Mittel, ein Verfahren zur Bekämpfung von Unkräutern und ein Verfahren zur Herstellung von herbiziden Mitteln

Die vorliegende Erfindung betrifft neue Oximether, mehrere Verfahren zu ihrer Herstellung sowie ihre Verwendung als Herbizide.

Es ist bereits bekannt geworden, daß man 2,6-Diethyl-N-methoxymethyl-chloracetanilid zur selektiven Unkrautbekämpfung verwenden kann (vgl. R. Wegler, Chemie der Pflanzenschutz- und Schädlingsbekämpfungsmittel, Band 5, Seite 255, Springer-Verlag (1977) sowie US-Patentschrift 3 442 945). Diese Verbindung ist jedoch nicht immer ausreichend wirksam und in ihrer Selektivität nicht immer ganz befriedigend.

Es wurden nun neue Oximether der Formel

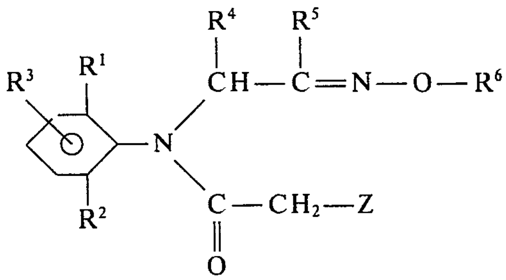

(I)

in welcher

$R^1$ für Wasserstoff, Alkyl oder Alkoxy steht,

$R^2$ für Wasserstoff, Alkyl, Alkoxy oder Halogen steht,

$R^3$ für Wasserstoff, Alkyl, Alkoxy oder Halogen steht,

$R^4$ für Wasserstoff oder Alkyl steht,

$R^5$ für Wasserstoff, Alkyl oder gegebenenfalls substituiertes Aryl steht,

$R^6$ für Wasserstoff, Alkyl, Alkenyl, Alkinyl, Alkoxyalkyl, Alkylthioalkyl, Dialkylaminoalkyl, Halogenalkyl, Alkoxycarbonylalkyl gegebenenfalls substituiertes Aryl oder gegebenenfalls substituiertes Aralkyl steht, und außerdem

$R^5$ und $R^6$gemeinsam für eine Alkylen-Gruppe stehen, und

Z für Halogen steht,

gefunden.

Die Verbindungen der Formel (I) können in der syn- oder anti-Form vorliegen; vorwiegend fallen sie als Gemische beider Formen an.

Weiterhin wurde gefunden, daß man die Oximether der Formel (I) erhält, wenn man

a) Anilinomethyloxim-ether der Formel

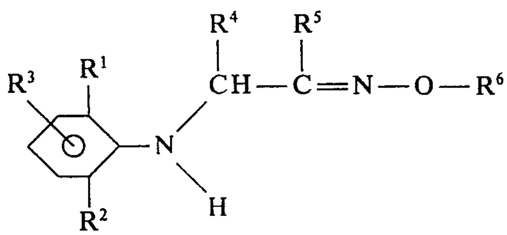

(II)

in welcher

$R^1$, $R^2$, $R^3$, $R^4$, $R^5$ und $R^6$ die oben angegebene Bedeutung haben,

mit Halogenessigsäurechloriden oder -bromiden bzw. -anhydriden der Formeln

$$Z—CH_2—CO—Cl(Br) \quad \text{(IIIa)}$$

bzw.

$$(Z—CH_2—CO)_2O \quad \text{(IIIb)}$$

in welchen

Z die oben angegebene Bedeutung hat,

in Gegenwart eines Verdünnungsmittels und gegebenenfalls in Gegenwart eines Säurebindemittels umsetzt, oder

b) Halogenacetanilide der Formel

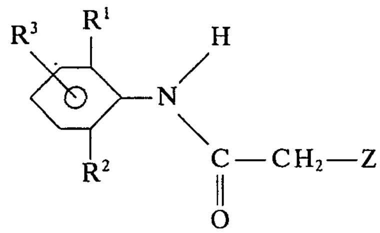

(IV)

in welcher
$R^1$, $R^2$, $R^3$ und Z die oben angegebene Bedeutung haben,
mit substituierten Oxim-ethern der Formel

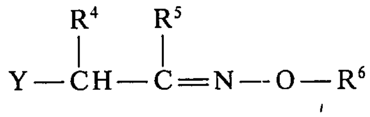

(V)

in welcher
$R^4$, $R^5$ und $R^6$ die oben angegebene Bedeutung haben, und
Y für Halogen, den Mesylat- oder Tosylat-Rest steht,
in Gegenwart eines Säurebinders und gegebenenfalls in Gegenwart eines Verdünnungsmittels umsetzt, oder

c) N-substituierte Halogenacetanilide der Formel

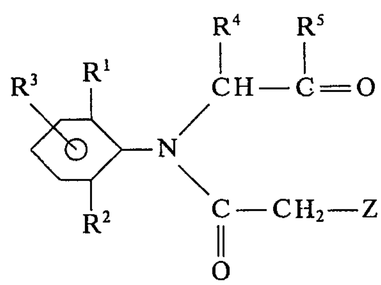

(VI)

in welcher
$R^1$, $R^2$, $R^3$, $R^4$, $R^5$ und Z die oben angegebene Bedeutung haben,
mit Salzen von Hydroxylamin-(Derivaten) der Formel

$H_2N—O—R^6$ (VII)

in welcher
$R^6$ die oben angegebene Bedeutung hat,
in Gegenwart eines Verdünnungsmittels und in Gegenwart eines Säurebindemittels umsetzt, oder

d) Alkalisalze von Oximen der Formel

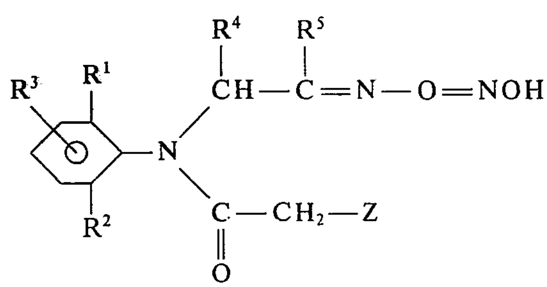

(VIII)

in welcher
$R^1$, $R^2$, $R^3$, $R^4$, $R^5$ und Z die oben angegebene Bedeutung haben,
mit Halogeniden der Formel

$X—R^7$ (IX)

in welcher

$R^7$ für Alkyl, Alkenyl, Alkinyl, Alkoxyalkyl, Alkylthioalkyl, Dialkylaminoalkyl, Halogenalkyl, Alkoxycarbonylalkyl, gegebenenfalls substituiertes Aryl oder gegebenenfalls substituiertes Aralkyl steht und

X für Chlor oder Brom steht,

in Gegenwart eines organischen Verdünnungsmittels oder in Gegenwart eines organisch-anorganischen Zweiphasensystems in Gegenwart eines Phasentransferkatalysators umsetzt, wobei die Alkalisalze der Oxime der Formel (VIII) in situ erzeugt werden.

Die neuen Oximether der Formel (I) weisen starke herbizide, insbesondere auch selektiv-herbizide Eigenschaften auf.

Überraschenderweise zeigen die erfindungsgemäßen Oximether bei sehr guter Unkrautwirkung insbesondere bessere Möglichkeiten zum Einsatz als selektive Unkrautbekämpfungsmittel in wichtigen Kulturpflanzen als das aus dem Stand der Technik bekannte 2,6-Diethyl-N-methoxymethyl-chloracetanilid, welches ein hochaktiver Wirkstoff gleicher Wirkungsart ist. Die erfindungsgemäßen Stoffe stellen somit eine Bereicherung der herbiziden Mittel zur selektiven Unkrautbekämpfung dar.

Die erfindungsgemäßen Oximether sind durch die Formel (I) allgemein definiert. In der Formel (I) steht $R^1$ vorzugsweise für Wasserstoff sowie geradkettiges oder verzweigtes Alkyl und Alkoxy mit jeweils 1 bis 4 Kohlenstoffatomen. $R^2$ und $R^3$ sind gleich oder verschieden und stehen vorzugsweise für Wasserstoff, geradkettiges oder verzweigtes Alkyl und Alkoxy mit jeweils 1 bis 4 Kohlenstoffatomen, sowie für die Halogene Fluor, Chlor und Brom. $R^4$ steht vorzugsweise für Wasserstoff und geradkettiges oder verzweigtes Alkyl mit 1 bis 4 Kohlenstoffatomen. $R^5$ steht vorzugsweise für Wasserstoff, geradkettiges oder verzweigtes Alkyl mit 1 bis 4 Kohlenstoffatomen, sowie für gegebenenfalls substituiertes Aryl mit 6 bis 10 Kohlenstoffatomen, wie insbesondere Phenyl und Naphthyl, wobei als Substituenten vorzugsweise in Frage kommen: Halogen, geradkettiges oder verzweigtes Alkyl mit 1 bis 4 Kohlenstoffatomen, Halogenalkyl mit 1 bis 2 Kohlenstoff- und 2 bis 5 Halogenatomen, wobei als Halogene vorzugsweise Fluor und Chlor genannt seien, Alkoxy und Alkylthio mit jeweils 1 oder 2 Kohlenstoffatomen, sowie Cyano und Nitro. $R^6$ steht vorzugsweise für Wasserstoff, geradkettiges oder verzweigtes Alkyl mit 1 bis 4 Kohlenstoffatomen, Alkenyl und Alkinyl mit jeweils 2 bis 4 Kohlenstoffatomen, Alkoxyalkyl und Alkylthioalkyl mit jeweils 1 bis 4 Kohlenstoffatomen im Alkylteil und 1 bis 4 Kohlenstoffatomen im Alkoxy- bzw. im Alkylthio-Teil, Dialkylaminoalkyl mit 1 bis 4 Kohlenstoffatomen in jedem Alkylteil, Halogenalkyl mit 1 bis 2 Kohlenstoff- und 1 bis 5 Halogenatomen, wobei als Halogene vorzugsweise Fluor und Chlor genannt seien, Alkoxycarbonylalkyl mit 1 bis 4 Kohlenstoffatomen im Alkylteil und 1 bis 4 Kohlenstoffatomen im Alkoxy-Teil, sowie für gegebenenfalls substituiertes Aryl und gegebenenfalls substituiertes Aralkyl mit 6 bis 10 Kohlenstoffatomen im Arylteil und 1 bis 2 Kohlenstoffatomen im Alkylteil, wie insbesondere Phenyl, Naphthyl und Benzyl, wobei als Substituenten vorzugsweise die bei $R^5$ bereits vorzugsweise genannten Arylsubstituenten in Frage kommen. $R^5$ und $R^6$ stehen außerdem gemeinsam vorzugsweise für eine zwei- bis viergliedrige Alkylen-Gruppe. Z steht vorzugsweise für die Halogene Chlor, Brom und Jod.

Ganz besonders bevorzugt sind diejenigen Oximether der Formel (I), in denen $R^1$, $R^2$ und $R^3$ gleich oder verschieden sind und für Wasserstoff, Methyl, Ethyl, Isopropyl, sek.-Butyl, tert.-Butyl, Methoxy, Ethoxy oder Isopropoxy stehen, $R^2$ und $R^3$ außerdem auch für Chlor oder Brom stehen; $R^4$ für Wasserstoff, Methyl oder Ethyl steht; $R^5$ für Wasserstoff, Methyl, Ethyl, Isopropyl sowie gegebenenfalls durch Chlor und/oder Methyl und/oder Methoxy und/oder Methylthio und/oder Trifluormethyl substituiertes Phenyl oder Naphthyl steht; $R^6$ für Wasserstoff, Methyl, Ethyl, Isopropyl, n-Propyl, n-Butyl, tert.-Butyl, Vinyl, Allyl, Propargyl, Methoxymethyl, Ethoxymethyl, Ethoxyethyl, Methylthiomethyl, Chlormethyl, Chlorethyl, Dimethylaminomethyl, Ethyl-methylaminomethyl, Diethylaminomethyl, Methoxycarbonylmethyl, gegebenenfalls durch Chlor und/oder Methyl und/oder Methoxy und/oder Methylthio und/oder Trifluormethyl substituiertes Phenyl, Naphthyl und Benzyl steht; $R^5$ und $R^6$ außerdem gemeinsam für die Di- und Trimethylen-Gruppe stehen; und Z für Chlor oder Brom steht.

Im einzelnen seien außer den bei den Herstellungsbeispielen genannten Verbindungen die folgenden Verbindungen der allgemeinen Formel (I) genannt:

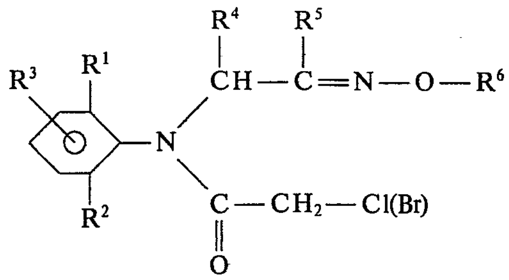

(I)

| $R^1$ | $R^2$ | $R^3$ | $R^4$ | $R^5$ | $R^6$ |
|---|---|---|---|---|---|
| H | H | H | H | H | H |
| $CH_3$ | $CH_3$ | H | H | H | H |
| $CH_3$ | $C_2H_5$ | H | H | H | H |
| $C_2H_5$ | $C_2H_5$ | H | H | H | H |
| $CH_3$ | $CH_3$ | H | H | H | $CH_3$ |
| $CH_3$ | $C_2H_5$ | H | H | H | $CH_3$ |
| $C_2H_5$ | $C_2H_5$ | H | H | H | $CH_3$ |
| $C_2H_5$ | H | H | H | H | $CH_3$ |
| $C(CH_3)_3$ | H | H | H | H | $CH_3$ |
| $CH_3$ | Cl | H | H | H | $CH_3$ |
| $CH_3$ | $O-CH_3$ | H | H | H | $CH_3$ |
| $CH_3$ | Br | H | H | H | $CH_3$ |
| $C(CH_3)_3$ | Cl | H | H | H | $CH_3$ |
| $OCH_3$ | $OCH_3$ | H | H | H | $CH_3$ |
| $CH_3$ | H | 3-$CH_3$ | H | H | $CH_3$ |
| i-$C_3H_7$ | $C_2H_5$ | H | H | H | $CH_3$ |
| $CH_3$ | H | H | H | H | $CH_3$ |
| $CH_3$ | $CH_3$ | H | H | H | $C_2H_5$ |
| $CH_3$ | $C_2H_5$ | H | H | H | $C_2H_5$ |
| $C_2H_5$ | $C_2H_5$ | H | H | H | $C_2H_5$ |
| $C_2H_5$ | H | H | H | H | $C_2H_5$ |
| $C(CH_3)_3$ | H | H | H | H | $C_2H_5$ |
| $CH_3$ | Cl | H | H | H | $C_2H_5$ |
| $CH_3$ | $O-CH_3$ | H | H | H | $C_2H_5$ |
| $CH_3$ | Br | H | H | H | $C_2H_5$ |
| $C(CH_3)_3$ | Cl | H | H | H | $C_2H_5$ |

Fortsetzung

| $R^1$ | $R^2$ | $R^3$ | $R^4$ | $R^5$ | $R^6$ |
|---|---|---|---|---|---|
| $OCH_3$ | $OCH_3$ | H | H | H | $C_2H_5$ |
| $CH_3$ | H | 3-$CH_3$ | H | H | $C_2H_5$ |
| i-$C_3H_7$ | $C_2H_5$ | H | H | H | $C_2H_5$ |
| $CH_3$ | H | H | H | H | $C_2H_5$ |
| $CH_3$ | $CH_3$ | H | H | H | n-$C_3H_7$ |
| $CH_3$ | $C_2H_5$ | H | H | H | n-$C_3H_7$ |
| $C_2H_5$ | $C_2H_5$ | H | H | H | n-$C_3H_7$ |
| $C(CH_3)_3$ | H | H | H | H | n-$C_3H_7$ |
| $CH_3$ | $CH_3$ | H | H | H | n-$C_4H_9$ |
| $CH_3$ | $C_2H_5$ | H | H | H | n-$C_4H_9$ |
| $C_2H_5$ | $C_2H_5$ | H | H | H | n-$C_4H_9$ |
| $C(CH_3)_3$ | H | H | H | H | n-$C_4H_9$ |
| $CH_3$ | $CH_3$ | H | H | H | t-$C_4H_9$ |
| $CH_3$ | $C_2H_5$ | H | H | H | t-$C_4H_9$ |
| $C_2H_5$ | $C_2H_5$ | H | H | H | t-$C_4H_9$ |
| $C(CH_3)_3$ | H | H | H | H | t-$C_4H_9$ |
| $CH_3$ | $CH_3$ | H | H | H | $-CH_2-C\equiv CH$ |
| $CH_3$ | $C_2H_5$ | H | H | H | $-CH_2-C\equiv CH$ |
| $C_2H_5$ | $C_2H_5$ | H | H | H | $-CH_2-C\equiv CH$ |
| $C(CH_3)_3$ | H | H | H | H | $-CH_2-C\equiv CH$ |
| $CH_3$ | $CH_3$ | H | H | H | $-CH_2-CH=CH_2$ |
| $CH_3$ | $C_2H_5$ | H | H | H | $-CH_2-CH=CH_2$ |
| $C_2H_5$ | $C_2H_5$ | H | H | H | $-CH_2-CH=CH_2$ |
| $C(CH_3)_3$ | H | H | H | H | $-CH_2-CH=CH_2$ |
| $CH_3$ | $CH_3$ | H | H | H | $C_6H_5$ |
| $CH_3$ | $C_2H_5$ | H | H | H | $C_6H_5$ |
| $C_2H_5$ | $C_2H_5$ | H | H | H | $C_6H_5$ |
| $C(CH_3)_3$ | H | H | H | H | $C_6H_5$ |
| $CH_3$ | $CH_3$ | H | H | H | $-CH_2-O-CH_3$ |
| $CH_3$ | $C_2H_5$ | H | H | H | $-CH_2-O-CH_3$ |
| $C_2H_5$ | $C_2H_5$ | H | H | H | $-CH_2-O-CH_3$ |

Fortsetzung

| $R^1$ | $R^2$ | $R^3$ | $R^4$ | $R^5$ | $R^6$ |
|---|---|---|---|---|---|
| $C(CH_3)_3$ | H | H | H | H | $-CH_2-O-CH_3$ |
| $CH_3$ | $CH_3$ | H | H | H | $-CH_2-S-CH_3$ |
| $CH_3$ | $C_2H_5$ | H | H | H | $-CH_2-S-CH_3$ |
| $C_2H_5$ | $C_2H_5$ | H | H | H | $-CH_2-S-CH_3$ |
| $C(CH_3)_3$ | H | H | H | H | $-CH_2-S-CH_3$ |
| $CH_3$ | $CH_3$ | H | H | H | $-CH_2-C_6H_5$ |
| $CH_3$ | $C_2H_5$ | H | H | H | $-CH_2-C_6H_5$ |
| $C_2H_5$ | $C_2H_5$ | H | H | H | $-CH_2-C_6H_5$ |
| $C(CH_3)_3$ | H | H | H | H | $-CH_2-C_6H_5$ |
| $CH_3$ | $CH_3$ | H | H | H | $-CH_2-CH_2Cl$ |
| $CH_3$ | $C_2H_5$ | H | H | H | $-CH_2-CH_2Cl$ |
| $C_2H_5$ | $C_2H_5$ | H | H | H | $-CH_2-CH_2Cl$ |
| $C(CH_3)_3$ | H | H | H | H | $-CH_2-CH_2Cl$ |
| $CH_3$ | $CH_3$ | H | H | $CH_3$ | n-$C_3H_7$ |
| $CH_3$ | $C_2H_5$ | H | H | $CH_3$ | n-$C_3H_7$ |
| $C_2H_5$ | $C_2H_5$ | H | H | $CH_3$ | n-$C_3H_7$ |
| $C(CH_3)_3$ | H | H | H | $CH_3$ | n-$C_3H_7$ |
| $CH_3$ | $CH_3$ | H | H | $CH_3$ | n-$C_4H_9$ |
| $CH_3$ | $C_2H_5$ | H | H | $CH_3$ | n-$C_4H_9$ |
| $C_2H_5$ | $C_2H_5$ | H | H | $CH_3$ | n-$C_4H_9$ |
| $C(CH_3)_3$ | H | H | H | $CH_3$ | n-$C_4H_9$ |
| $CH_3$ | $CH_3$ | H | H | $CH_3$ | t-$C_4H_9$ |
| $CH_3$ | $C_2H_5$ | H | H | $CH_3$ | t-$C_4H_9$ |
| $C_2H_5$ | $C_2H_5$ | H | H | $CH_3$ | t-$C_4H_9$ |
| $C(CH_3)_3$ | H | H | H | $CH_3$ | t-$C_4H_9$ |
| $CH_3$ | $CH_3$ | H | H | $CH_3$ | $-CH_2-C\equiv CH$ |
| $CH_3$ | $C_2H_5$ | H | H | $CH_3$ | $-CH_2-C\equiv CH$ |
| $C_2H_5$ | $C_2H_5$ | H | H | $CH_3$ | $-CH_2-C\equiv CH$ |
| $C(CH_3)_3$ | H | H | H | $CH_3$ | $-CH_2-C\equiv CH$ |
| $CH_3$ | $CH_3$ | H | H | $CH_3$ | $-CH_2-CH=CH_2$ |
| $CH_3$ | $C_2H_5$ | H | H | $CH_3$ | $-CH_2-CH=CH_2$ |

Fortsetzung

| $R^1$ | $R^2$ | $R^3$ | $R^4$ | $R^5$ | $R^6$ |
|---|---|---|---|---|---|
| $C_2H_5$ | $C_2H_5$ | H | H | $CH_3$ | $-CH_2-CH=CH_2$ |
| $C(CH_3)_3$ | H | H | H | $CH_3$ | $-CH_2-CH=CH_2$ |
| $CH_3$ | $CH_3$ | H | H | $CH_3$ | $C_6H_5$ |
| $CH_3$ | $C_2H_5$ | H | H | $CH_3$ | $C_6H_5$ |
| $C_2H_5$ | $C_2H_5$ | H | H | $CH_3$ | $C_6H_5$ |
| $C(CH_3)_3$ | H | H | H | $CH_3$ | $C_6H_5$ |
| $CH_3$ | $CH_3$ | H | H | $CH_3$ | $-CH_2-O-CH_3$ |
| $CH_3$ | $C_2H_5$ | H | H | $CH_3$ | $-CH_2-O-CH_3$ |
| $C_2H_5$ | $C_2H_5$ | H | H | $CH_3$ | $-CH_2-O-CH_3$ |
| $C(CH_3)_3$ | H | H | H | $CH_3$ | $-CH_2-O-CH_3$ |
| $CH_3$ | $CH_3$ | H | H | $CH_3$ | $-CH_2-S-CH_3$ |
| $CH_3$ | $C_2H_5$ | H | H | $CH_3$ | $-CH_2-S-CH_3$ |
| $C_2H_5$ | $C_2H_5$ | H | H | $CH_3$ | $-CH_2-S-CH_3$ |
| $C(CH_3)_3$ | H | H | H | $CH_3$ | $-CH_2-S-CH_3$ |
| $CH_3$ | $CH_3$ | H | H | $CH_3$ | $-CH_2-C_6H_5$ |
| $CH_3$ | $C_2H_5$ | H | H | $CH_3$ | $-CH_2-C_6H_5$ |
| $C_2H_5$ | $C_2H_5$ | H | H | $CH_3$ | $-CH_2-C_6H_5$ |
| $C(CH_3)_3$ | H | H | H | $CH_3$ | $-CH_2-C_6H_5$ |
| $CH_3$ | $CH_3$ | H | H | $CH_3$ | $-CH_2-CH_2-Cl$ |
| $CH_3$ | $C_2H_5$ | H | H | $CH_3$ | $-CH_2-CH_2-Cl$ |
| $C_2H_5$ | $C_2H_5$ | H | H | $CH_3$ | $-CH_2-CH_2-Cl$ |
| $C(CH_3)_3$ | H | H | H | $CH_3$ | $-CH_2-CH_2-Cl$ |
| $CH_3$ | $CH_3$ | H | H | | $-(CH_2)_2-$ |
| $CH_3$ | $C_2H_5$ | H | H | | $-(CH_2)_2-$ |
| $C_2H_5$ | $C_2H_5$ | H | H | | $-(CH_2)_2-$ |
| $C(CH_3)_3$ | H | H | H | | $-(CH_2)_2-$ |
| $CH_3$ | $CH_3$ | H | H | | $-(CH_2)_3-$ |
| $CH_3$ | $C_2H_5$ | H | H | | $-(CH_2)_3-$ |
| $C_2H_5$ | $C_2H_5$ | H | H | | $-(CH_2)_3-$ |
| $C(CH_3)_3$ | H | H | H | | $-(CH_2)_3-$ |
| $CH_3$ | $CH_3$ | H | $CH_3$ | | $-(CH_2)_2-$ |

Fortsetzung

| $R^1$ | $R^2$ | $R^3$ | $R^4$ | $R^5$ | $R^6$ |
|---|---|---|---|---|---|
| $CH_3$ | $C_2H_5$ | H | $CH_3$ | | $-(CH_2)_2-$ |
| $C_2H_5$ | $C_2H_5$ | H | $CH_3$ | | $-(CH_2)_2-$ |
| $C(CH_3)_3$ | H | H | $CH_3$ | | $-(CH_2)_2-$ |
| $CH_3$ | $CH_3$ | H | $CH_3$ | | $-(CH_2)_3-$ |
| $CH_3$ | $C_2H_5$ | H | $CH_3$ | | $-(CH_2)_3-$ |
| $C_2H_5$ | $C_2H_5$ | H | $CH_3$ | | $-(CH_2)_3-$ |
| $C(CH_3)_3$ | H | H | $CH_3$ | | $-(CH_2)_3-$ |
| $CH_3$ | $CH_3$ | H | H | $C_2H_5$ | H |
| $CH_3$ | $C_2H_5$ | H | H | $C_2H_5$ | H |
| $C_2H_5$ | $C_2H_5$ | H | H | $C_2H_5$ | H |
| $C(CH_3)_3$ | H | H | H | $C_2H_5$ | H |
| $CH_3$ | $CH_3$ | H | H | $C_2H_5$ | $CH_3$ |
| $CH_3$ | $C_2H_5$ | H | H | $C_2H_5$ | $CH_3$ |
| $C_2H_5$ | $C_2H_5$ | H | H | $C_2H_5$ | $CH_3$ |
| $C(CH_3)_3$ | H | H | H | $C_2H_5$ | $CH_3$ |
| $CH_3$ | $CH_3$ | H | H | $C_2H_5$ | $C_2H_5$ |
| $CH_3$ | $C_2H_5$ | H | H | $C_2H_5$ | $C_2H_5$ |
| $C_2H_5$ | $C_2H_5$ | H | H | $C_2H_5$ | $C_2H_5$ |
| $C(CH_3)_3$ | H | H | H | $C_2H_5$ | $C_2H_5$ |
| $CH_3$ | $CH_3$ | H | H | $C_6H_5$ | H |
| $CH_3$ | $C_2H_5$ | H | H | $C_6H_5$ | H |
| $C_2H_5$ | $C_2H_5$ | H | H | $C_6H_5$ | H |
| $CH_3$ | $CH_3$ | H | H | $C_6H_5$ | $CH_3$ |
| $CH_3$ | $C_2H_5$ | H | H | $C_6H_5$ | $CH_3$ |
| $C_2H_5$ | $C_2H_5$ | H | H | $C_6H_5$ | $CH_3$ |
| $C(CH_3)_3$ | H | H | H | $C_6H_5$ | $CH_3$ |
| $CH_3$ | Cl | H | H | $C_6H_5$ | $CH_3$ |
| $CH_3$ | Br | H | H | $C_6H_5$ | $CH_3$ |
| $OCH_3$ | $CH_3$ | H | H | $C_6H_5$ | $CH_3$ |
| $CH_3$ | H | H | H | $C_6H_5$ | $CH_3$ |
| $CH_3$ | H | 3-CH | H | $C_6H_5$ | $CH_3$ |

Fortsetzung

| $R^1$ | $R^2$ | $R^3$ | $R^4$ | $R^5$ | $R^6$ |
|---|---|---|---|---|---|
| $CH_3$ | H | 5-$CH_3$ | H | $C_6H_5$ | $CH_3$ |
| $CH_3$ | $CH_3$ | H | H | $C_6H_5$ | $C_2H_5$ |
| $CH_3$ | $C_2H_5$ | H | H | $C_6H_5$ | $C_2H_5$ |
| $C_2H_5$ | $C_2H_5$ | H | H | $C_6H_5$ | $C_2H_5$ |
| $C(CH_3)_3$ | H | H | H | $C_6H_5$ | $C_2H_5$ |
| $CH_3$ | $CH_3$ | H | H | $C_6H_5$ | n-$C_3H_7$ |
| $CH_3$ | $C_2H_5$ | H | H | $C_6H_5$ | n-$C_3H_7$ |
| $C_2H_5$ | $C_2H_5$ | H | H | $C_6H_5$ | n-$C_3H_7$ |
| $C(CH_3)_3$ | H | H | H | $C_6H_5$ | n-$C_3H_7$ |
| $CH_3$ | $CH_3$ | H | $CH_3$ | H | $CH_3$ |
| $CH_3$ | $C_2H_5$ | H | $CH_3$ | H | $CH_3$ |
| $C_2H_5$ | $C_2H_5$ | H | $CH_3$ | H | $CH_3$ |
| $C(CH_3)_3$ | H | H | $CH_3$ | H | $CH_3$ |
| $CH_3$ | $CH_3$ | H | $CH_3$ | $CH_3$ | $CH_3$ |
| $CH_3$ | $C_2H_5$ | H | $CH_3$ | $CH_3$ | $CH_3$ |
| $C_2H_5$ | $C_2H_5$ | H | $CH_3$ | $CH_3$ | $CH_3$ |
| $C(CH_3)_3$ | H | H | $CH_3$ | $CH_3$ | $CH_3$ |
| $CH_3$ | $CH_3$ | H | $CH_3$ | H | $C_2H_5$ |
| $CH_3$ | $C_2H_5$ | H | $CH_3$ | H | $C_2H_5$ |
| $C_2H_5$ | $C_2H_5$ | H | $CH_3$ | H | $C_2H_5$ |
| $C(CH_3)_3$ | H | H | $CH_3$ | H | $C_2H_5$ |
| $CH_3$ | $CH_3$ | H | $CH_3$ | $CH_3$ | $C_2H_5$ |
| $CH_3$ | $C_2H_5$ | H | $CH_3$ | $CH_3$ | $C_2H_5$ |
| $C_2H_5$ | $C_2H_5$ | H | $CH_3$ | $CH_3$ | $C_2H_5$ |
| $C(CH_3)_3$ | H | H | $CH_3$ | $CH_3$ | $C_2H_5$ |
| $CH_3$ | $CH_3$ | H | $CH_3$ | H | n-$C_3H_7$ |
| $CH_3$ | $C_2H_5$ | H | $CH_3$ | H | n-$C_3H_7$ |
| $C_2H_5$ | $C_2H_5$ | H | $CH_3$ | H | n-$C_3H_7$ |
| $C(CH_3)_3$ | H | H | $CH_3$ | H | n-$C_3H_7$ |
| $CH_3$ | $CH_3$ | H | $CH_3$ | $CH_3$ | n-$C_3H_7$ |
| $CH_3$ | $C_2H_5$ | H | $CH_3$ | $CH_3$ | n-$C_3H_7$ |

Fortsetzung

| $R^1$ | $R^2$ | $R^3$ | $R^4$ | $R^5$ | $R^6$ |
|---|---|---|---|---|---|
| $C_2H_5$ | $C_2H_5$ | H | $CH_3$ | $CH_3$ | n-$C_3H_7$ |
| $C(CH_3)_3$ | H | H | $CH_3$ | $CH_3$ | n-$C_3H_7$ |
| $CH_3$ | $CH_3$ | H | $CH_3$ | H | n-$C_4H_9$ |
| $CH_3$ | $C_2H_5$ | H | $CH_3$ | H | n-$C_4H_9$ |
| $C_2H_5$ | $C_2H_5$ | H | $CH_3$ | H | n-$C_4H_9$ |
| $C(CH_3)_3$ | H | H | $CH_3$ | H | n-$C_4H_9$ |

Verwendet man 2,6-Dimethylanilinomethyl-methylketoximmethylether und Chloracetylchlorid als Ausgangsstoffe, so kann der Reaktionsablauf durch das folgende Formelschema wiedergegeben werden (Verfahrensvariante a):

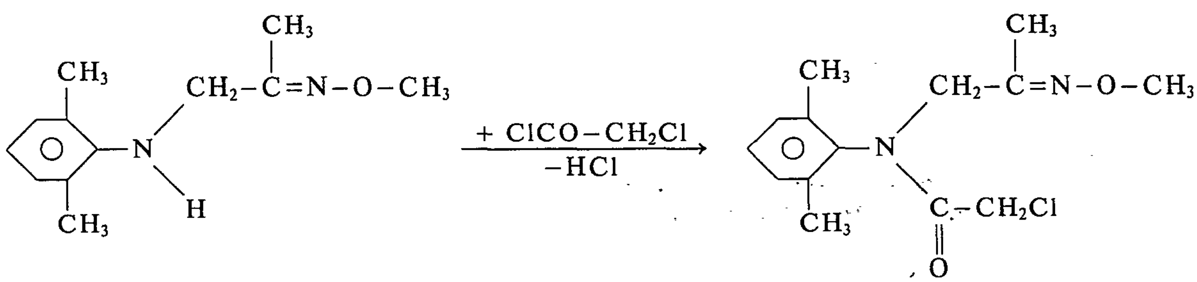


Verwendet man 2-Ethyl-6-methyl-chloracetanilid und Chlor-methylaldoxim-methylether als Ausgangsstoffe, so kann der Reaktionsablauf durch das folgende Formelschema wiedergegeben werden (Verfahrensvariante b):

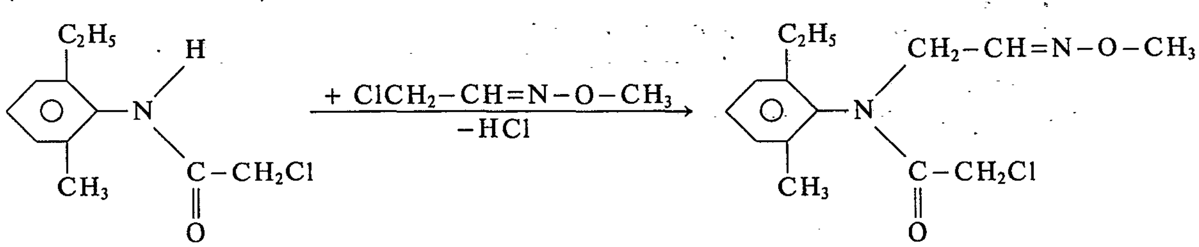


Verwendet man 2,6-Dimethyl-N-acetylmethyl-chloracetanilid und O-Methyl-hydroxylamin-hydrochlorid als Ausgangsstoffe, so kann der Reaktionsablauf durch das folgende Formelschema wiedergegeben werden (Verfahrensvariante c):

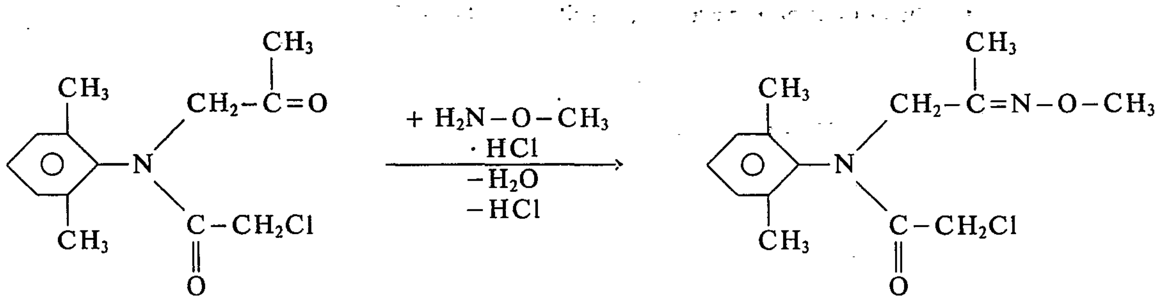


Verwendet man das Natriumsalz von 2,6-Dimethyl-chloracet-anilidomethyl-methylketoxim und Benzylchlorid als Ausgangsstoffe, so kann der Reaktionsablauf durch das folgende Formelschema wiedergegeben werden (Verfahrensvariante d):

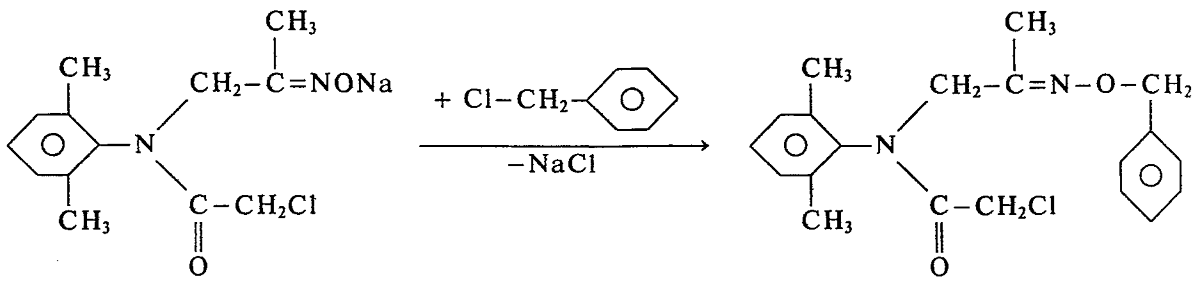


Die bei der Durchführung des erfindungsgemäßen Verfahrens (a) als Ausgangsstoffe benötigten Anilino-ketoxim-ether sind durch die Formel (II) allgemein definiert. In dieser Formel stehen $R^1$ bis $R^6$ vorzugsweise für diejenigen Reste, die bereits im Zusammenhang mit der Beschreibung der erfindungsgemäßen Stoffe der Formel (I) vorzugsweise für diese Reste genannt wurden.

Die Anilinomethyloxim-ether der Formel (II) sind noch nicht bekannt. Man erhält

e) Verbindungen der Formel (II) wenn man Aniline der Formel

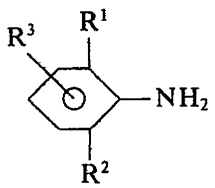

(X)

in welcher

$R^1$, $R^2$ und $R^3$ die oben angegebene Bedeutung haben,

α) mit substituierten Oxim-ethern der Formel

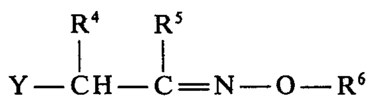

(V)

in welcher

$R^4$, $R^5$, $R^6$ und Y die oben angegebene Bedeutung haben,

in Gegenwart eines Säurebinders und gegebenenfalls in Gegenwart eines Verdünnungsmittels umsetzt, oder

β) mit Propargylhalogeniden der Formel

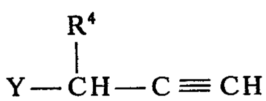

(XI)

in welcher

$R^4$ und Y die oben angegebene Bedeutung haben,

in Gegenwart eines Säurebinders und gegebenenfalls in Gegenwart eines Verdünnungsmittels umsetzt, die entstehenden Propargyl-aniline der Formel

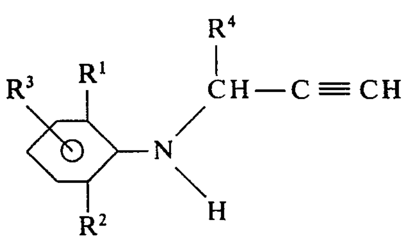

(XII)

in welcher

$R^1$, $R^2$, $R^3$ und $R^4$ die oben angegebene Bedeutung haben,

in üblicher Weise hydratisiert und die entstehenden Anilin-Derivate der Formel

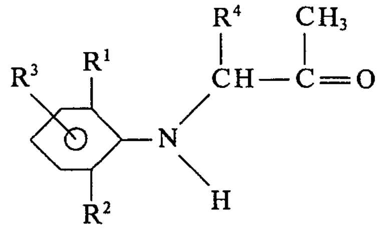

(XIII)

in welcher
$R^1$, $R^2$, $R^3$ und $R^4$ die oben angegebene Bedeutung haben,
mit Salzen von Hydroxylamin-(Derivaten) der Formel

$$H_2N—O—R^6 \qquad (VII)$$

in welcher
$R^6$ die oben angegebene Bedeutung hat,
in Gegenwart eines Verdünnungsmittels und in Gegenwart eines Säurebindemittels umsetzt,
oder

γ) mit Keto-Derivaten der Formel

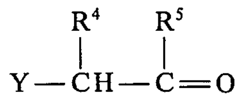

(XIV)

in welcher
$R^4$, $R^5$ und Y die oben angegebene Bedeutung haben,
in Gegenwart eines Säurebinders und gegebenenfalls in Gegenwart eines Verdünnungsmittels umsetzt, und die entstehenden substituierten Anilin-Derivate der Formel

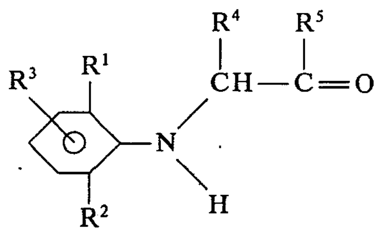

(XV)

in welcher
$R^1$, $R^2$, $R^3$, $R^4$ und $R^5$ die oben angegebene Bedeutung haben,
mit Salzen von Hydroxylamin-(Derivaten) der Formel

$$H_2N—O—R^6 \qquad (VII)$$

in welcher
$R^6$ die oben angegebene Bedeutung hat,
in Gegenwart eines Verdünnungsmittels und in Gegenwart eines Säurebindemittels umsetzt.
oder

f) diejenigen Verbindungen der Formel (II), in denen $R^6$ nicht für Wasserstoff steht, wenn man Anilin-Derivate der Formel

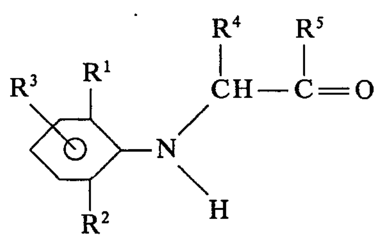

(XV)

in welcher
$R^1$, $R^2$, $R^3$, $R^4$ und $R^5$ die oben angegebene Bedeutung haben,
mit Salzen des Hydroxylamins in Gegenwart eines Verdünnungsmittels und in Gegenwart eines Säurebindemittels umsetzt und die dabei entstehenden Oxime der Formel

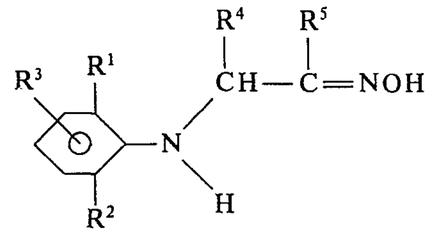

(XVI)

in welcher
$R^1$, $R^2$, $R^3$, $R^4$ und $R^5$ die oben angegebene Bedeutung haben,
in Form ihrer Alkali-Salze mit Dimethylsulfat oder mit Halogeniden der Formel

$$X—R^7 \qquad (IX)$$

in welcher
$R^7$ und X die oben angegebene Bdeutung haben,
in Gegenwart eines organischen Verdünnungsmittels oder in Gegenwart eines organisch-anorganischen Zweiphasensystems in Gegenwart eines Phasentransferkatalysators umsetzt, wobei die Alkalisalze der Oxime der Formel (XVI) in situ erzeugt werden.

Die bei der Herstellung der Anilinomethyloxim-ether der Formel (II) als Ausgangsstoffe benötigten Aniline der Formel (X) sind allgemein bekannte Verbindungen der organischen Chemie. Als Beispiele seien genannt:

Anilin; 2-Methylanilin; 2-Ethylanilin; 2-Isopropylanilin; 2-sek.-Butylanilin; 2-tert.-Butylanilin; 2,6-Dimethylanilin; 2,3-Dimethylanilin; 2,5-Dimethylanilin; 2,6-Diethylanilin; 2-Ethyl-6-methylanilin; 2,4,6-Trimethylanilin; 2-Ethyl-4,6-dimethylanilin; 2,6-Diethyl-4-methylanilin; 2,6-Diisopropyl-4-methylanilin; 2,3,6-Trimethylanilin; 2-Methyl-6-chloranlin; 2-tert.-Butyl-6-chloranilin; 2-Methoxy-6-methylanilin; 2,6-Dimethoxyanilin; 2-Methoxy-6-ethylanilin; 2,6-Diethoxyanilin.

Die bei der Herstellung der Anilinomethyloxim-ether der Formel (II) nach dem Verfahren (e) (Variante α) weiterhin als Ausgangsstoffe benötigten substituierten Oximether der Formel (V) sind bekannt (vgl. z. B. US-Patentschrift 3 896 189); ebenso die für das Verfahren (e), Variante (β) benötigten Propargylhalogenide der Formel (XI) (vgl. Houben-Weyl, Methoden der organischen Chemie, Band V/3 und 4); sowie auch die für das Verfahren (e), Variante (γ) benötigten Keto-Derivate der Formel (XIV) (vgl. Houben-Weyl, Methoden der organischen Chemie, Band VII/2 a, b, c) und die Hydroxylamin-(Derivate) der Formel (VII) (vgl. Houben-Weyl, Methoden der organischen Chemie, Band X/1); bzw. lassen sich die jeweiligen Verbindungen nach den in den erwähnten Literaturstellen angegebenen Verfahren leicht herstellen.

Bei der Herstellung der Anilinomethyloxim-ether der Formel (II) nach dem Verfahren (e), Varianten (α), (β) und (γ) können als Säurebindemittel alle üblichen Säureakzeptoren verwendet werden. Vorzugsweise in Betracht kommen Alkalicarbonate, wie Kalium- oder Natriumcarbonat.

Als Verdünnungsmittel können bei dem Verfahren (e), Varianten (α), (β) und (γ) alle üblichen inerten organischen Lösungsmittel eingesetzt werden. Vorzugsweise in Betracht kommen aromatische Lösungsmittel, wie insbesondere Toluol, sowie Dimethylformamid. In der zweiten Stufe des Verfahrens (e), Variante (γ) kommen vorzugsweise Alkohole, wie insbesondere Methanol, als Lösungsmittel in Frage.

Die Reaktionstemperaturen können bei dem Verfahren (e), Varianten (α), (β) und (γ) innerhalb eines größeren Bereiches variiert werden. Im allgemeinen arbeitet man zwischen 0°C und 180°C, vorzugsweise zwischen 20°C und 160°C.

Bei der Umsetzung gemäß Verfahren (e), Varianten (α), (β) und (γ) arbeitet man vorzugsweise mit äquimolaren Mengen. Es ist jedoch auch möglich, eine der Komponenten, vorzugsweise das Anilin der Formel (X), in einem Überschuß einzusetzen. Die Aufarbeitung und Isolierung der Reaktionsprodukte erfolgt nach üblichen Methoden.

Die bei der Durchführung des Verfahrens (f) als Ausgangsstoffe benötigten Oxime der Formel (XVI) sind bisher noch nicht bekannt. Sie lassen sich jedoch in einfacher Weise nach dem Verfahren (e), Variante (γ) herstellen.

Die bei dem Verfahren (f) weiterhin als Ausgangsstoffe benötigten Halogenide sind durch die Formel (IX) allgemein definiert. In dieser Formel haben $R^7$ und X vorzugsweise diejenigen Bedeutungen, die im Zusammenhang mit der Beschreibung des erfindungsgemäßen Verfahrens (d) vorzugsweise für diese Reste genannt werden.

Bei dem Verfahren (f) kommen als Verdünnungsmittel alle inerten organischen Lösungsmittel in Frage. Hierzu gehören vorzugsweise Ether, wie Diethylether und Dioxan; aromatische Kohlenwasserstoffe, wie Toluol und Benzol; in einzelnen Fällen auch chlorierte Kohlenwasserstoffe, wie Chloroform,

Methylenchlorid und Tetrachlorkohlenstoff; sowie Hexamethylphosphorsäuretriamid.

Die Reaktionstemperaturen können bei der Durchführung des Verfahrens (f) in einem größeren Bereich variiert werden. Im allgemeinen arbeitet man zwischen 20 und 150°C, vorzugsweise bei Raumtemperatur. In einzelnen Fällen ist es vorteilhaft, bei der Siedetemperatur des Lösungsmittels, beispielsweise zwischen 60 und 100°C, zu arbeiten.

Bei der Durchführung des Verfahrens (f) setzt man auf 1 Mol Alkalisalz eines Oxims der Formel (XVI) vorzugsweise 1 bis 3 Mol Halogen der Formel (IX) ein. Die Aufarbeitung und Isolierung der Reaktionsprodukte erfolgt nach üblichen Methoden.

In einer bevorzugten Ausführungsform des Verfahrens (f) wird zweckmäßigerweise so verfahren, daß man von einem Oxim der Formel (XVI) ausgeht, letzteres in einem geeigneten inerten organischen Lösungsmittel mittels Alkalimetall-hydrid oder -amid in das Salz überführt, und letzteres ohne Isolierung sofort mit einem Halogenid der Formel (IV) umsetzt, wobei unter Austritt von Alkalihalogenid die Verbindungen der Formel (II) in einem Arbeitsgang erhalten werden.

Nach einer weiteren bevorzugten Ausführungsform des Verfahrens (f) werden zweckmäßigerweise die Herstellung der Salze der Oxime der Formel (XVI) sowie die Umsetzung in einem Zweiphasensystem, wie beispielsweise wäßrige Natron- und Kalilauge/Toluol oder Methylenchlorid, unter Zusatz von 0,01—1 Mol eines Phase-Transfer-Katalysators, wie beispielsweise Ammonium- oder Phosphoniumverbindungen, durchgeführt.

Die außerdem für das erfindungsgemäße Verfahren (a) als Ausgangsstoffe zu verwendenden Halogenessigsäurechloride und -bromide bzw. -anhydride der Formeln (IIIa) und (IIIb) sind allgemein bekannte Verbindungen der organischen Chemie. Als Beispiele seien genannt: Chloracetylchlorid, Bromacetylchlorid, Jodacetylchlorid und die entsprechenden Bromide sowie Anhydride.

Die bei der Durchführung des erfindungsgemäßen Verfahrens (b) als Ausgangsstoffe benötigten Halogenacetanilide sind durch die Formel (IV) allgemein definiert. In dieser Formel stehen $R^1$, $R^2$, $R^3$ und Z vorzugsweise für diejenigen Reste, die bereits im Zusammenhang mit der Beschreibung der erfindungsgemäßen Stoffe der Formel (I) vorzugsweise für diese Reste genannt wurden.

Die Halogenacetanilide der Formel (IV) sind allgemein bekannt bzw. können sie in allgemein bekannter Art und Weise erhalten werden, indem man entsprechende Aniline mit einem Halogenessigsäurechlorid oder -bromid bzw. -anhydrid der Formeln (IIIa) bzw. (IIIb) in Gegenwart eines inerten organischen Lösungsmittels, wie beispielsweise Toluol oder Dimethylformamid, gegebenenfalls in Gegenwart eines Säurebindemittels, wie z. B. Kaliumcarbonat oder Triethylamin, bei Temperaturen zwischen 0 und 100°C umsetzt. Als Beispiele seien die Chlor- und Bromacetanilide der oben genannten Aniline der Formel (X) genannt.

Die außerdem für das erfindungsgemäße Verfahren (b) als Ausgangsstoffe zu verwendenden substituierten Oxim-ether sind durch die Formel (V) allgemein definiert. In dieser Formel stehen $R^4$, $R^5$ und $R^6$ vorzugsweise für diejenigen Reste, die bereits im Zusammenhang mit der Beschreibung der erfindungsgemäßen Stoffe der Formel (I) vorzugsweise genannt wurden. Y steht vorzugsweise für Chlor, Brom, den Mesylat- und Tosylat-Rest.

Die substituierten Oxim-ether der Formel (V) sind bekannt (vgl. z. B. US-Patentschrift 3 896 189); bzw. können sie in allgemein bekannter Art und Weise erhalten werden, indem man entsprechende Carbonylverbindungen mit Hydroxylamin-(Derivaten) in Gegenwart eines Lösungsmittels, vorzugsweise eines Alkohols bzw. eines wäßrigen Alkohols, bei Temperaturen zwischen 20°C und 100°C, vorzugsweise zwischen 50°C und 80°C umsetzt. Dabei wird das Hydroxylamin-(Derivat) vorzugsweise in Form eines Salzes, insbesondere als Hydrochlorid, gegebenenfalls in Gegenwart eines Säurebindemittels, wie beispielsweise Natriumacetat, eingesetzt. Die Isolierung der Endprodukte erfolgt in üblicher Weise.

Die bei der Durchführung des erfindungsgemäßen Verfahrens (c) als Ausgangsstoffe benötigten N-substituierten Halogenacetanilide sind durch die Formel (VI) allgemein definiert. In dieser Formel stehen $R^1$, $R^2$, $R^3$, $R^4$ $R^5$ und Z vorzugsweise für diejenigen Reste, die bereits im Zusammenhang mit der Beschreibung der erfindungsgemäßen Stoffe der Formel (I) vorzugsweise genannt wurden.

Die N-substituierten Halogenacetanilide der Formel (VI) sind zum Teil bekannt (DE-OS 2 726 253). Sie lassen sich in einfacher Weise nach mehreren Verfahren herstellen. So erhält man N-substituierte Halogenacetanilide der Formel (VI), wenn man

g) Halogenacetanilide der Formel

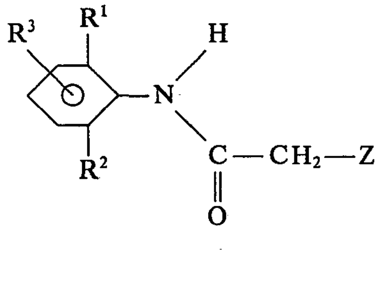

(IV)

in welcher
$R^1$, $R^2$, $R^3$ und Z die oben angegebene Bedeutung haben,
α) mit Keto-Derivaten der Formel

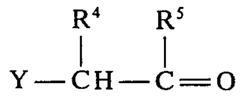

(XIV)

in welcher
$R^4$, $R^5$ und Y die oben angegebene Bedeutung haben,
in Gegenwart eines Säurebinders und gegebenenfalls in Gegenwart eines Verdünnungsmittels umsetzt, oder
β) mit Propargylhalogeniden der Formel

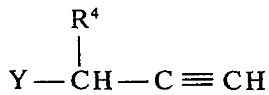

(XI)

in welcher
$R^4$ und Y die oben angegebene Bedeutung haben,
in Gegenwart eines Säurebinders und gegebenenfalls in Gegenwart eines Verdünnungsmittels umsetzt und die entstehenden Propargyl-halogenide der Formel

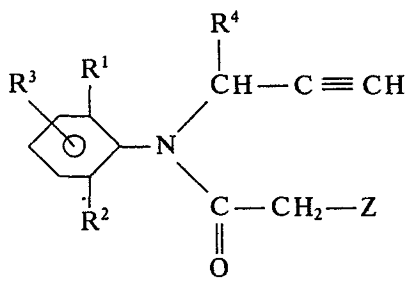

(XVII)

in welcher
$R^1$, $R^2$, $R^3$, $R^4$ und Y die oben angegebene Bedeutung haben,
in üblicher Weise hydratisiert, oder
h) Anilin-Derivate der Formel

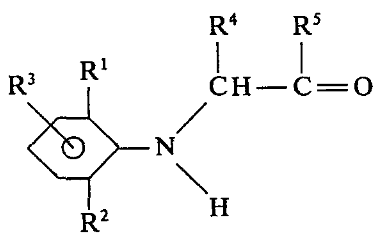

(XV)

in welcher
$R^1$, $R^2$, $R^3$, $R^4$ und $R^5$ die oben angegebene Bedeutung haben,
mit bekannten Halogenessigsäurechloriden oder -bromiden der Formel

$$Z-CH_2-CO-Cl(Br) \qquad (IIIa)$$

in welcher
Z die oben angegebene Bedeutung hat,
in Gegenwart eines Verdünnungsmittels und gegebenenfalls in Gegenwart eines Säurebindemittels umsetzt.

Die bei der Herstellung der N-substituierten Halogenacetanilide der Formel (VI) als Ausgangsstoffe benötigten Anilin-Derivate der Formel (XV) können entsprechend Verfahren (e), Variante (γ, erste Stufe), erhalten werden.

Bei der Herstellung der N-substituierten Halogenacetanilide der Formel (VI) nach den Verfahren (g) und (h) können als Säurebindemittel alle üblichen Säureakzeptoren verwendet werden. Vorzugsweise in Betracht kommen Alkalicarbonate, wie Kalium- oder Natriumcarbonat.

Als Verdünnungsmittel können bei den Verfahren (g), Varianten ($\alpha$) und ($\beta$), und (h) alle üblichen inerten organischen Lösungsmittel eingesetzt werden. Vorzugsweise in Betracht kommen aromatische Lösungsmittel, wie insbesondere Toluol, sowie Dimethylformamid.

Nach einer bevorzugten Ausführungsform wird die Umsetzung gemäß Verfahren (h) in einem Zweiphasensystem, wie beispielsweise wäßrige Natron- oder Kalilauge/Toluol oder Methylenchlorid, gegebenenfalls unter Zusatz von 0,1—1 Mol eines Phasen-transfer-Katalysators, wie beispielsweise einer Ammonium- oder Phosphoniumverbindung, durchgeführt.

Die Reaktionstemperaturen können bei den Verfahren (g), Varianten ($\alpha$) und ($\beta$) und (h) innerhalb eines größeren Bereiches variiert werden. Im allgemeinen arbeitet man zwischen 0°C und 180°C, vorzugsweise zwischen 20°C und 160°C.

Bei der Umsetzung gemäß den Verfahren (g), Varianten ($\alpha$) und ($\beta$), sowie Verfahren (h) arbeitet man vorzugsweise mit äquimolaren Mengen. Die Aufarbeitung und Isolierung der Reaktionsprodukte erfolgt nach üblichen Methoden.

Die außerdem für das erfindungsgemäße Verfahren (c) als Ausgangsstoffe (in Form ihrer Salze) zu verwendenden Hydroxylamin-(Derivate) sind durch die Formel (VII) allgemein definiert. In dieser Formel steht $R^6$ vorzugsweise für diejenigen Reste, die bereits im Zusammenhang mit der Beschreibung der erfindungsgemäßen Stoffe der Formel (I) vorzugsweise genannt wurden. Die Verbindungen der Formel (VII) werden vorzugsweise in Form ihrer Hydrohalogenide, wie insbesondere als Hydrochlorid, eingesetzt.

Die bei der Durchführung des erfindungsgemäßen Verfahrens (d) als Ausgangsstoffe benötigten Halogenide sind durch die Formel (IX) allgemein definiert. In dieser Formel steht $R^7$ vorzugsweise für geradkettiges oder verzweigtes Alkyl mit 1 bis 4 Kohlenstoffatomen, Alkenyl und Alkinyl mit jeweils 2 bis 4 Kohlenstoffatomen, Alkoxyalkyl und Alkylthioalkyl mit jeweils 1 bis 4 Kohlenstoffatomen im Alkylteil und 1 bis 4 Kohlenstoffatomen im Alkoxy- bzw. im Alkylthio-Teil, Dialkylaminoalkyl mit 1 bis 4 Kohlenstoffatomen in jeden Alkylteil, Halogenalkyl mit 1 bis 2 Kohlenstoffatomen und 1 bis 5 Halogenatomen, wobei als Halogene vorzugsweise Fluor und Chlor genannt seien, Alkoxycarbonylalkyl mit 1 bis 4 Kohlenstoffatomen im Alkylteil und 1 bis 4 Kohlenstoffatomen im Alkoxyteil, sowie für gegebenenfalls substituiertes Aryl und gegebenenfalls substituiertes Aralkyl mit jeweils 6 bis 10 Kohlenstoffatomen im Arylteil und 1 bis 2 Kohlenstoffatomen im Alkylteil, wobei als Aryl- bzw. Aralkylreste speziell Phenyl, Naphthyl und Benzyl genannt seien, und als Substituenten vorzugsweise die bei $R^5$ bereits vorzugsweise genannten Arylsubstituenten in Frage kommen.

Die Hydroxylamin-(Derivate) der Formel (VII) und die Halogenide der Formel (IX) sind allgemein bekannte Verbindungen der organischen Chemie.

Als Verdünnungsmittel kommen für die erfindungsgemäße Umsetzung gemäß Verfahren (a) vorzugsweise inerte organische Lösungsmittel infrage. Hierzu gehören vorzugsweise Ketone, wie Diethylketon, insbesondere Aceton und Methylethylketon; Nitrile, wie Propionitril, insbesondere Acetonitril; Ether, wie Tetrahydrofuran oder Dioxan; aliphatische und aromatische Kohlenwasserstoffe, wie Petrolether, Benzol, Toluol oder Xylol; halogenierte Kohlenwasserstoffe, wie Methylenchlorid, Tetrachlorkohlenstoff, Chloroform oder Chlorbenzol; und Ester, wie Essigester.

Das erfindungsgemäße Verfahren (a) kann gegebenenfalls in Gegenwart von Säurebindern (Halogenwasserstoff-Akzeptoren) durchgeführt werden. Als solche können alle üblichen säurebindemittel verwendet werden. Hierzu gehören vorzugsweise organische Basen, wie tertiäre Amine, beispielsweise Triethylamin, oder Pyridin, ferner anorganische Basen, wie beispielsweise Alkalihydroxide und Alkalicarbonate.

Die Reaktionstemperaturen können bei der Durchführung des erfindungsgemäßen Verfahrens (a) in einem größeren Bereich variiert werden. Im allgemeinen arbeitet man zwischen 0°C und 120°C, vorzugsweise zwischen 0 und 100°C.

Bei der Durchführung des erfindungsgmäßen Verfahrens (a) setzt man vorzugsweise auf 1 Mol der Verbindungen der Formel (II) 1 bis 1,5 Mol Halogenacetylierungsmittel und 1 bis 1,5 Mol Säurebinder ein. Die Isolierung der Verbindungen der Formel (I) erfolgt in üblicher Weise.

Als Verdünnungsmittel kommen für die erfindungsgemäße Umsetzung gemäß Verfahren (b) vorzugsweise inerte organische Lösungsmittel infrage. Hierzu gehören vorzugsweise die bei dem Verfahren (a) bereits genannten Solventien.

Als Säurebindemittel kommen bei dem Verfahren (b) vorzugsweise die bei dem Verfahren (a) bereits genannten Stoffe infrage.

Die Reaktionstemperaturen können bei der Durchführung des erfindungsgemäßen Verfahrens (b) in einem größeren Bereich variiert werden. Im allgemeinen arbeitet man zwischen 0°C und 150°C, vorzugsweise zwischen 20°C und 100°C.

Bei der Durchführung des erfindungsgemäßen Verfahrens (b) arbeitet man vorzugsweise mit äquimolaren Mengen. Es ist jedoch auch möglich, eine der Komponenten in einem Überschuß einzusetzen. Die Aufarbeitung und Isolierung der Reaktionsprodukte erfolgt nach üblichen Methoden.

In einer bevorzugten Ausführungsform wird die erfindungsgemäße Umsetzung gemäß Verfahren (b) in einem Zweiphasensystem, wie beispielsweise wäßrige Natron- oder Kalilauge/Toluol oder Methylenchlorid, gegebenenfalls unter Zusatz von 0,1—1 Mol eines Phasen-transfer-Katalysators, wie beispielsweise einer Ammonium- oder Phosphoniumverbindung, beispielsweise seien Benzyl-dodecyl-dimethyl-ammoniumchlorid (Zephirol) und Triethyl-benzyl-ammoniumchlorid genannt, durchgeführt (vergleiche auch die Herstellungsbeispiele).

Als Verdünnungsmittel kommen für die erfindungsgemäße Umsetzung gemäß Verfahren (c) vorzugsweise Alkohole bzw. wäßrige Alkohole infrage.

Da die Verbindungen der Formel (VII) bei der Durchführung des Verfahrens (c) in Form ihrer Salze eingesetzt werden, vorzugsweise als Hydrochloride, wird in Gegenwart eines Säurebindemittels gearbeitet. Hierzu gehören vorzugsweise Alkalicarbonate und -acetate.

Die Reaktionstemperaturen können bei der Durchführung des erfindungsgemäßen Verfahrens (c) in einem größeren Berich variiert werden. Im allgemeinen arbeitet man zwischen 20 und 120°C, vorzugsweise zwischen 40°C und 100°C.

Bei der Durchführung des erfindungsgemäßen Verfahrens (c) arbeitet man vorzugsweise mit äquimolaren Mengen. Es ist jedoch auch möglich, eine der Komponenten in einem Überschuß einzusetzen. Die Aufarbeitung und Isolierung der Reaktionsprodukte erfolgt nach üblichen Methoden.

Als Verdünnungsmittel kommen für die erfindungsgemäße Umsetzung gemäß Verfahren (d) alle inerten organischen Lösungsmittel infrage. Hierzu gehören vorzugsweise Ether, wie Diethylether und Dioxan; aromatische Kohlenwasserstoffe, wie Toluol und Benzol; in einzelnen Fällen auch chlorierte Kohlenwasserstoffe, wie Chloroform, Methylenchlorid und Tetrachlorkohlenstoff; sowie Hexamethylphosphorsäure-triamid.

Die Reaktionstempe ıturen können bei der Durchführung des erfindungsgemäßen Verfahrens (d) in einem größeren Bere n variiert werden. Im allgemeinen arbeitet man zwischen 20 und 150°C, vorzugsweise bei Raumtemperatur. In einzelnen Fällen ist es vorteilhaft, bei der Siedetemperatur des Lösungsmittels, beispielsweise zwischen 60 und 100°C, zu arbeiten.

Bei der Durchführung des erfindungsgemäßen Verfahrens (d) setzt man auf 1 Mol Alkalisalz eines Oxims der Formel (VIII) vorzugsweise 1 bis 3 Mol Halogenid der Formel (IX) ein. Die Aufarbeitung und Isolierung der Reaktionsprodukte erfolgt nach üblichen Methoden.

In einer bevorzugten Ausführungsform des Verfahrens (d) wird zweckmäßigerweise so verfahren, daß man von einem Oxim der Formel (VIII) ausgeht, letzteres in einem geeigneten inerten organischen Lösungsmittel mittels Alkalimetall-hydrid oder -amid in das Salz überführt, und letzteres ohne Isolierung sofort mit einem Halogenid der Formel (IV) umsetzt, wobei unter Austritt von Alkalihalogenid die erfindungsgemäßen Verbindungen der Formel (I) in einem Arbeitsgang erhalten werden.

Nach einer weiteren bevorzugten Ausführungsform des Verfahrens (d) werden zweckmäßigerweise die Herstellung der Salze der Oxime der Formel (VIII) sowie die erfindungsgemäße Umsetzung in einem Zweiphasensystem, wie beispielsweise wäßrige Natron- und Kalilauge/Toluol oder Methylenchlorid, unter Zusatz von 0,01—1 Mol eines Phasen-Transfer-Katalysators, wie beispielsweise einer Ammonium- oder Phosphoniumverbindung, durchgeführt.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden:

Dikotyle Unkräuter der Gattungen:
: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen:
: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

Monokotyle Unkräuter der Gattungen:
: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:
: Oryza, Zea, Triticum, Rordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z. B. auf Industrie- und Gleisanlagen und auf Wegen und Plätzen mit und ohne Baumbewuchs. Ebenso können die Verbindungen zur Unkrautbekämpfung in Dauerkulturen z. B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe zeigen insbesondere neben einer sehr guten Wirkung gegen grasartige Unkräuter und gegen Cyperus-Arten auch eine gute herbizide Wirkung bei breitblättrigen Unkräutern. Ein selektiver Einsatz der erfindungsgemäßen Wirkstoffe ist möglich, vorzugsweise in Getreide, Baumwolle und Zuckerrüben, oder auch in anderen Kulturen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsionskonzentrate, Wirkstoff-imprägnierte Natur- und synthetische Stoffe, Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln, und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzol, Chlorethylen oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser.

Als feste Trägerstoffe kommen in Frage: z. B. natürliche Gesteinsmehle, wie Kaoline, Tonerden, Talkum, Kreide, Quarz, Attapilgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Ether, z. B. Alkylarylpolyglykol-ether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserunsmitteln ist möglich.

Die Wirkstoffe können als solche, in Form ihrerer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Streuen.

Die erfindungsgemäßen Wirkstoffe können sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,1 und 10 kg Wirkstoff pro ha, vorzugsweise zwischen 0,1 und 5 kg/ha.

Die erfindungsgemäßen Wirkstoffe zeigen bei Nachlaufaufanwendung wachstumsregulierende Eigenschaften.

Wirkung und Einsatzmöglichkeiten zur Unkrautbekämpfung gehen aus dem Beispiel A hervor.

In dem nachfolgenden Beispiel wird die nachstehend angegebene Verbindung als Vergleichssubstanz eingesetzt:

A = 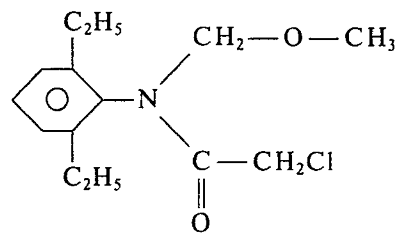

2,6-Diethyl-N-methoxymethyl-chloracetanilid

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

Wirkstoffe, Aufwandmengen und Resultate werden ermittelt.

Der erfindungsgemäße Wirkstoff des Beispiels 1 zeigt in diesem Test eine bessere selektive Wirksamkeit als die aus dem Stand der Technik bekannte Substanz (A).

Tabelle

Beispiel A
Pre-emergence-Test

| Wirkstoff | Wirkstoffaufwand kg/ha | Echinochloa | Setaria | Sinapis | Matricaria | Galinsoga | Stellaria | Lolium | Weizen | Baumwolle | Rüben |
|---|---|---|---|---|---|---|---|---|---|---|---|
| (A) | 5,0 | 100 | 100 | 20 | 90 | 100 | 90 | 90 | 60 | 20 | 60 |
| (bekannt) | 2,5 | 90 | 100 | 0 | 90 | 100 | 90 | 90 | 60 | 0 | 50 |
| (1) | 5,0 | 100 | 100 | 40 | 90 | 100 | 80 | 100 | 60 | 0 | 0 |
| | 2,5 | 80 | 100 | 0 | 90 | 100 | 80 | 100 | 60 | 0 | 0 |

Herstellungsbeispiele

Beispiel 1

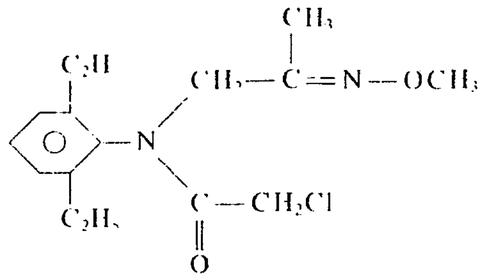

(Verfahren (c))

Ein Gemisch aus 14,1 g (0,05 Mol) 2,6-Diethyl-N-(2'-oxo)-propyl-chloracetanilid, 4,2 g (0,05 Mol) O-Methyl-hydroxylammoniumchlorid und 7 g (0,05 Mol) gepulvertem Kaliumcarbonat wird bei 20° C 24 Stunden gerührt. Danach wird das Reaktionsgemisch durch Abdestillieren des Lösungsmittels im Vakuum eingeengt. Der Rückstand wird zwischen Wasser und Methylenchlorid verteilt, die organische Phase abgetrennt, über Natriumsulfat getrocknet und eingeengt. Der kristalline Rückstand wird bei −10° C aus Petrolether umkristallisiert. Man erhält 8,5 g (56% der Theorie) 2,6-Diethyl-N-(2'-methoxy-imino)-propyl-chloracetanilid vom Schmelzpunkt 56—57° C.

Herstellung der Vorstufen

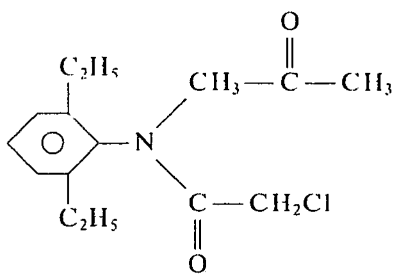

(VI-1)

(Verfahren (g) Variante (β))

39,5 g (0,15 Mol) 2,6-Diethyl-N-propargyl-chloracetanilid werden portionsweise bei 80° C in 85%ige Ameisensäure eingetragen. Nach zweistündigem Rühren bei 80° C wird die Reaktionsmischung auf gesättigte Amonsulfatlösung gegossen, ausgeethert und mit Natriumcarbonat neutralisiert. Nach dem Trocknen über Natriumsulfat wird im Vakuum eingeengt und der Rückstand durch Anreiben mit Petrolether zur Kristallisation gebracht. Man erhält 35,7 g (85% der Theorie) 2,6-Diethyl-N-(2'-oxo)-propyl-chloracetanilid vom Schmelzpunkt 65—67° C.

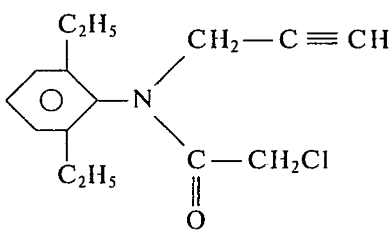

112,7 g (0,5 Mol) 2,6-Diethyl-chloracetanilid werden in einem Zwei-Phasengemisch aus 500 ml Methylenchlorid und 250 ml 50%iger Natronlauge/Wasser nach Zusatz von 1,5 g Triethylbenzylammonium-chlorid (TEBA) schnell gerührt und bei 25—35° C tropfenweise mit 59,5 g (0,5 Mol) Propargylbromid versetzt. Es wird noch vier Stunden bei 20° C gerührt, die organische Phase abgetrennt, mehrmals mit Wasser gewaschen, über Natriumsulfat getrocknet und im Vakuum eingeengt. Der Rückstand wird aus Isopropanol umkristallisiert. Man erhält 67,8 g (51,5% der Theorie) 2,6-Diethyl-N-propargyl-chloracetanilid vom Schmelzpunkt 57—58° C.

## Beispiel 2

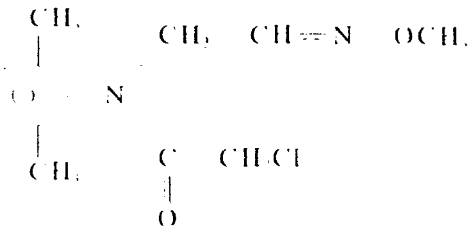

(Verfahren (a))

6 g (0,03 Mol) 2,6-Dimethyl-N-(2'-methoxyimino-ethyl)-anilin und 2,8 g (0,035 Mol) Pyridin werden in 80 ml wasserfreiem Tetrahydrofuran zum Sieden erhitzt und mit 4 g (0,035 Mol) Chloracetylchlorid versetzt. Nach 15 Minuten Erhitzen unter Rückfluß wird die Reaktionsmischung abgekühlt und im Vakuum eingeengt. Der Rückstand wird zwischen Wasser und Methylenchlorid verteilt, die organische Phase abgetrennt, über Natriumsulfat getrocknet und eingeengt. Der ölige Rückstand kristallisiert nach Behandeln mit Petrolether. Man erhält 4,8 g (60% der Theorie) 2,6-Dimethyl-N-(2'-methoxyimino-ethyl)-chloracetanilid vom Schmelzpunkt 56—58° C.

### Herstellung des Ausgangsproduktes

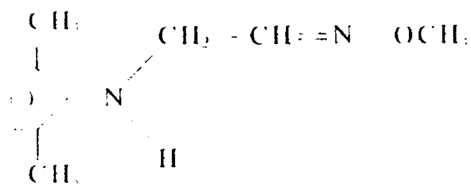

(II-1)

(Verfahren (e), Variante (α))

48,4 g (0,4 Mol) 2,6-Dimethylanilin und 27,6 g (0,2 Mol) fein gepulvertes Kaliumcarbonat werden in 80 ml Dimethylformamid unter Rühren auf 100°C erhitzt und tropfenweise mit 21,5 g (0,2 Mol) Chloracetaldehyd-O-methyl-oximether versetzt. Nach 3 Stunden Rühren bei 100°C wird das anorganische Salz abfiltriert. Das Filtrat wird mit 250 ml Methylenchlorid versetzt, mehrmals mit Wasser ausgeschüttelt, über Natriumsulfat getrocknet und im Vakuum eingeengt. Der Rückstand wird destilliert. Man erhält 14,2 g (37% der Theorie) 2,6-Dimethyl-N-(2'-methoxy-imino-ethyl)-anilin vom Siedepunkt 105—110°C/0,133 mbar und vom Brechungsindex n : 1,537

## Beispiel 3

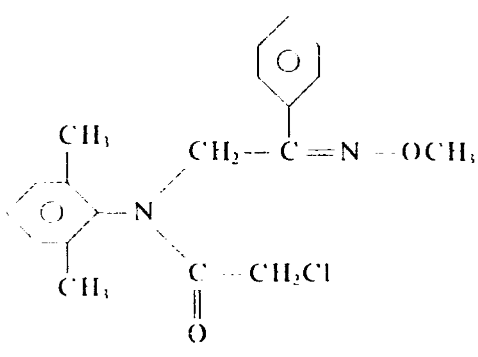

(Verfahren (a))

13,4 g (0,05 Mol) α-(2,6-Dimethylphenylamino)-acetophenon-oxim-O-methylether werden in 200 ml Toluol gelöst, mit 4 ml (0,05 Mol) Chloracetylchlorid versetzt und 6 Stunden bis zur beendeten Gasenteicklung unter Rückfluß erhitzt. Man läßt abkühlen und engt durch Abdestillieren des

Lösungsmittels im Vakuum ein. Der Rückstand wird chromatographisch gereinigt. Man erhält 3,1 g (18% der Theorie) α-[2,6-Dimethylphenyl)-N-chloracetyl-amino]-acetophenon-oxim-O-methylether vom Schmelzpunkt 80° C.

Herstellung der Vorstufen

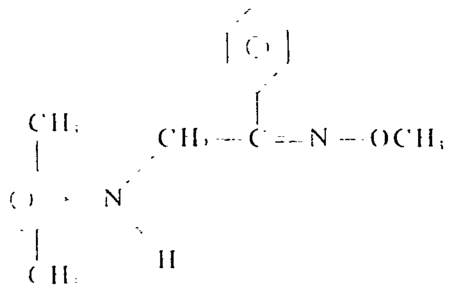

(II-2)

(Verfahren (f))

31,2 g (0,11 Mol) α-(2,6-Dimethylphenylamino)acetophenon-oxim-hydrochlorid und 0,6 g (0,176 Mol) Benzyldimethylammoniumchlorid werden in 110 ml Methylenchlorid gelöst und nach Zugabe von 110 ml konzentrierter Natronlauge und 6,9 g (0,055 Mol) Dimethylsulfat 6 Tage bei 20° C gerührt. Nach Aufgießen auf Eis wird die organische Phase abgetrennt, mit Wasser gewaschen, über Natriumsulfat getrocknet und eingedampft. Man erhält 25,7 g (87% der Theorie) α-(2,6-Dimethylphenylamino)-acetophenonoxim-O-methylether vom Brechungsindex n : 1,5877.

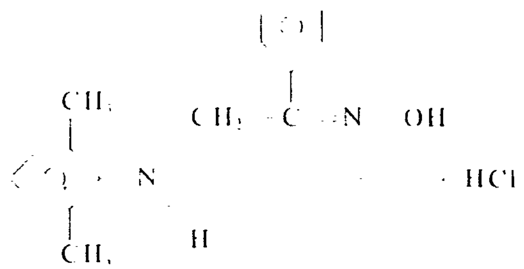

(II-3)

(Verfahren (e) Variante (γ))

59,7 g (0,3 Mol) Phenacylbromid (Herstellung vgl. Org. Syntheses Vol. II, S. 480) und 73,2 g (0,6 Mol) 2,6-Dimethylanilin werden in 100 ml Ethylalkohol 10 Minuten auf 80° C erhitzt und danach im Vakuum eingeengt. Der Rückstand wird in Toluol aufgenommen und filtriert. Das Filtrat wird mit Ethylalkohol verdünnt und mit 20,8 g (0,165 Mol) Hydroxylammoniumchlorid sowie mit 22,6 g (0,165 Mol) gepulvertem Kaliumcarbonat versetzt. Nach 20stündigem Rühren bei Raumtemperatur wird filtriert und das Filtrat eingeengt. Der Rückstand wird durch Verreiben mit wenig Ethylalkohol zur Kristallisation gebracht. Man erhält 33,9 g (40% der Theorie) α-(2,6-Dimethylphenylamino)-acetophenonoxim-hydrochlorid vom Schmelzpunkt 200° C.

Nach einem oder mehreren der in der Anmeldung beschriebenen Verfahren und entsprechend den Beispielen 1 bis 3 werden die in der nachstehenden Tabelle 1 formelmäßig aufgeführten erfindungsgemäßen Verbindungen der Formel (I) erhalten.

Tabelle 1

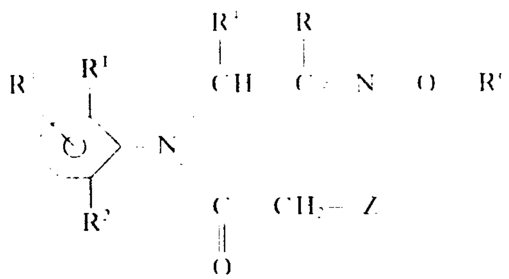

(I)

| Beispiel Nr. | $R^1$ | $R^2$ | $R^3$ | $R^4$ | $R^5$ | $R^6$ | Z | Schmelzpunkt (°C) bzw. Brechungsindex |
|---|---|---|---|---|---|---|---|---|
| 4 | $CH_3$ | $CH_3$ | H | H | $CH_3$ | $CH_3$ | Cl | 61–75 |
| 5 | $CH_3$ | $C_2H_5$ | H | H | $CH_3$ | $CH_3$ | Cl | 53 |
| 6 | $CH_3$ | $CH_3$ | H | H | $CH_3$ | $C_2H_5$ | Cl | 70–82 |
| 7 | $C_2H_5$ | $CH_3$ | H | H | $CH_3$ | $C_2H_5$ | Cl | 62 |
| 8 | $C_2H_5$ | $C_2H_5$ | H | H | $CH_3$ | n-$C_4H_9$ | Cl | $n_D^{23}$ : 1,520 |
| 9 | $CH_3$ | $C_2H_5$ | H | H | $CH_3$ | n-$C_4H_9$ | Cl | $n_D^{23}$ : 1,523 |
| 10 | $C_2H_5$ | $C_2H_5$ | H | H | H | $CH_3$ | Cl | 49–50 |
| 11 | $CH_3$ | $C_2H_5$ | H | H | H | $CH_3$ | Cl | $n_D^{23}$ : 1,541 |
| 12 | $C_2H_5$ | $C_2H_5$ | H | H | H | $C_2H_5$ | Cl | Kristallbrei |
| 13 | $CH_3$ | $C_2H_5$ | H | H | H | $C_2H_5$ | Cl | $n_D^{23}$ : 1,5336 |
| 14 | $C_2H_5$ | $C_2H_5$ | H | H | $CH_3$ | H | Cl | 139–40 |
| 15 | $CH_3$ | $C_2H_5$ | H | H | $CH_3$ | H | Cl | 111–13 |
| 16 | $C_2H_5$ | $C_2H_5$ | H | H | H | n-$C_4H_9$ | Cl | $n_D^{23}$ : 1,521 |
| 17 | $CH_3$ | Cl | H | H | $CH_3$ | $CH_3$ | Cl | 77–78 |
| 18 | H | $CH_3$ | H | H | $CH_3$ | $CH_3$ | Cl | $n_D^{20}$ = 1,5402 |
| 19 | H | $C(CH_3)_3$ | H | H | $CH_3$ | $CH_3$ | Cl | 95–101 |
| 20 | $CH_3$ | $CH_3$ | H | H | $CH_3$ | $C_3H_7$ | Cl | Kristallbrei |
| 21 | $CH_3$ | H | 3-$CH_3$ | H | $CH_3$ | $CH_3$ | Cl | 48–52 |
| 22 | $C_2H_5$ | $C_2H_5$ | H | H | $CH_3$ | $C_2H_5$ | Cl | $n_D^{20}$ = 1,5282 |
| 23 | $CH_3$ | $C_2H_5$ | H | H | $CH_3$ | $C_3H_7$ | Cl | $n_D^{20}$ = 1,5276 |
| 24 | $CH_3$ | $CH_3$ | H | H | $CH_3$ | H | Cl | 123–125 |
| 25 | $CH_3$ | $CH_3$ | H | $CH_3$ | $CH_3$ | $CH_3$ | Cl | $n_D^{20}$ = 1,5388 |
| 26 | $CH_3$ | $CH_3$ | H | $CH_3$ | H | $CH_3$ | Cl | $n_D^{20}$ = 1,5398 |
| 27 | $CH_3$ | $CH_3$ | H | H | H | $C_2H_5$ | Cl | $n_D^{20}$ = 1,5369 |
| 28 | $CH_3$ | $C_2H_5$ | H | $CH_3$ | $CH_3$ | $CH_3$ | Cl | $n_D^{20}$ = 1,5298 |
| 29 | $C_2H_5$ | $C_2H_5$ | H | H | $CH_3$ | $C_3H_7$ | Cl | $n_D^{20}$ = 1,5262 |
| 30 | $C_2H_5$ | $C_2H_5$ | H | $CH_3$ | $CH_3$ | $CH_3$ | Cl | $n_D^{20}$ = 1,5300 |
| 31 | $CH_3$ | $CH_3$ | H | H | H | $C_3H_7$ | Cl | $n_D^{20}$ = 1,5312 |
| 32 | $CH_3$ | $C_2H_5$ | H | H | H | $C_3H_7$ | Cl | $n_D^{20}$ = 1,5155 |

Fortsetzung

| Beispiel Nr. | $R^1$ | $R^2$ | $R^3$ | $R^4$ | $R^5$ | $R^6$ | Z | Schmelzpunkt (°C) bzw. Brechungsindex |
|---|---|---|---|---|---|---|---|---|
| 33 | $C_2H_5$ | $C_2H_5$ | H | H | H | $C_3H_7$ | Cl | $n_D^{20} = 1{,}5209$ |
| 34 | $CH_3$ | $CH_3$ | H | $CH_3$ | H | $C_2H_5$ | Cl | $n_D^{20} = 1{,}5348$ |
| 35 | $CH_3$ | $C_2H_5$ | H | $CH_3$ | H | $CH_3$ | Cl | Kristallbrei |
| 36 | $CH_3$ | $C_2H_5$ | H | $CH_3$ | H | $C_2H_5$ | Cl | $n_D^{20} = 1{,}5290$ |
| 37 | $C_2H_5$ | $C_2H_5$ | H | $CH_3$ | H | $C_2H_5$ | Cl | $n_D^{20} = 1{,}5273$ |
| 38 | $CH_3$ | $CH_3$ | H | $CH_3$ | $CH_3$ | $C_2H_5$ | Cl | $n_D^{20} = 1{,}5296$ |
| 39 | $CH_3$ | $C_2H_5$ | H | $CH_3$ | $CH_3$ | $C_2H_5$ | Cl | $n_D^{20} = 1{,}5288$ |
| 40 | $CH_3$ | $CH_3$ | H | $C_2H_5$ | H | $CH_3$ | Cl | $n_D^{20} = 1{,}5372$ |
| 41 | $CH_3$ | $CH_3$ | H | H | $CH_3$ | $C_4H_9$ | Cl | $n_D^{20} = 1{,}5299$ |

Nach einem oder mehreren der in der Anmeldung beschriebenen Verfahren und entsprechend den Beispielen 2 und 3 werden die in der nachstehenden Tabelle 2 formelmäßig aufgeführten Ausgangsprodukte der Formel (II) erhalten

Tabelle 2

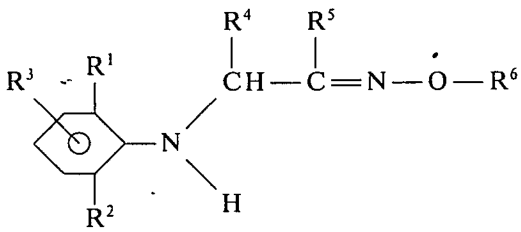

(II)

| Bsp. Nr. | $R^1$ | $R^2$ | $R^3$ | $R^4$ | $R^5$ | $R^6$ | Kp(°C)/mbar bzw. $n_D^{20}$ |
|---|---|---|---|---|---|---|---|
| (II-4) | $C_2H_5$ | $CH_3$ | H | H | H | $CH_3$ | 120–125/0,133<br>1,531 |
| (II-5) | $C_2H_5$ | $C_2H_5$ | H | H | H | $CH_3$ | 112–117/0,133<br>1,5295 |
| (II-6) | $CH_3$ | $C_2H_5$ | H | H | H | $C_2H_5$ | 120–121/0,133<br>1,525 |
| (II-7) | $C_2H_5$ | $C_2H_5$ | H | H | H | $C_2H_5$ | 135–140/0,133<br>1,525 |
| (II-8) | $C_2H_5$ | $C_2H_5$ | H | H | H | n-$C_4H_9$ | 120–135/0,133<br>1,513 |

Nach einem oder mehreren der in der Anmeldung beschriebenen Verfahren und entsprechend Beispiel 1 werden die in der nachstehenden Tabelle 3 formelmäßig aufgeführten Ausgangsprodukte der Formel (VI) erhalten.

Tabelle 3

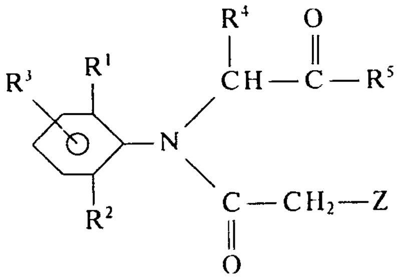

(VI)

| Bsp. Nr. | $R^1$ | $R^2$ | $R^3$ | $R^4$ | $R^5$ | Z | Schmelzpunkt (°C) |
|---|---|---|---|---|---|---|---|
| (VI-2) | $CH_3$ | $CH_3$ | H | H | $CH_3$ | Cl | 95 |
| (VI-3) | $CH_3$ | $C_2H_5$ | H | H | $CH_3$ | Cl | 48–49 |
| (VI-4) | $CH_3$ | $CH_3$ | H | H | 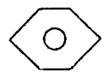 | Cl | 100 |
| (VI-5) | $CH_3$ | $CH_3$ | H | $CH_3$ | $CH_3$ | Cl | 104 |
| (VI-6) | $CH_3$ | $C_2H_5$ | H | H | $C(CH_3)_3$ | Cl | 86 |
| (VI-7) | $CH_3$ | $CH_3$ | H | H | 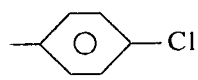 | Cl | 82 |
| (VI-8) | $C_2H_5$ | $C_2H_5$ | H | H | 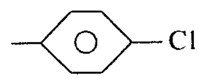 | Cl | 100 |
| (VI-9) | $CH_3$ | $CH_3$ | H | H | 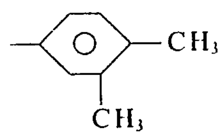 | Cl | 90 |
| (VI-10) | $CH_3$ | $CH_3$ | H | H | 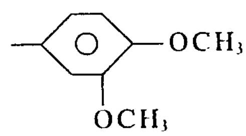 | Cl | 114 |
| (VI-11) | $CH_3$ | $CH_3$ | H | H | 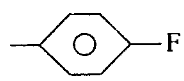 | Cl | 104 |
| (VI-12) | $CH_3$ | $CH_3$ | H | 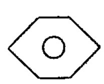 | 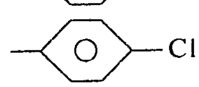 | Cl | 149 |
| (VI-13) | $CH_3$ | $CH_3$ | H | $CH_3$ | 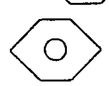 | Cl | 84 |

**Patentansprüche**

1. Oximether der Formel

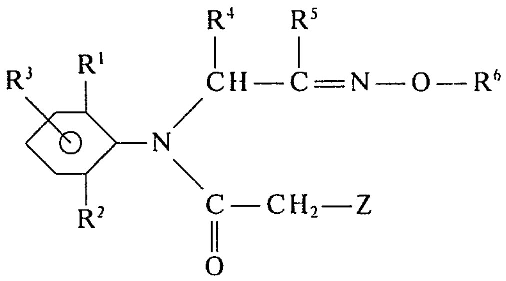

(I)

in welcher
$R^1$ für Wasserstoff, Alkyl oder Alkoxy steht,
$R^2$ für Wasserstoff, Alkyl, Alkoxy oder Halogen steht,
$R^3$ für Wasserstoff, Alkyl, Alkoxy oder Halogen steht,
$R^4$ für Wasserstoff oder Alkyl steht,
$R^5$ für Wasserstoff, Alkyl oder gegebenenfalls substituiertes Aryl steht,
$R^6$ für Wasserstoff, Alkyl, Alkenyl, Alkinyl, Alkoxyalkyl, Alkylthioalkyl, Dialkylaminoalkyl, Halogenalkyl, Alkoxycarbonylalkyl, gegebenenfalls substituiertes Aryl oder gegebenenfalls substituiertes Aralkyl steht, und außerdem
$R^5$ und $R^6$ gemeinsam für eine Alkylen-Gruppe stehen, und
Z für Halogen steht.

2. Verfahren zur Herstellung von Oximethern der Formel

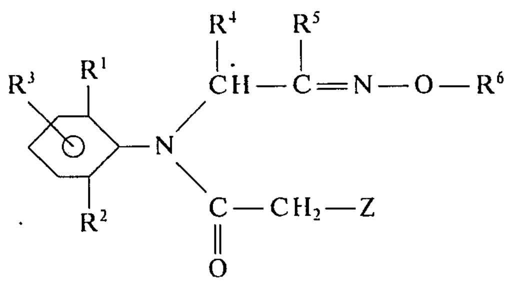

(I)

in welcher
$R^1$ für Wasserstoff, Alkyl oder Alkoxy steht,
$R^2$ für Wasserstoff, Alkyl, Alkoxy oder Halogen steht,
$R^3$ für Wasserstoff, Alkyl, Alkoxy oder Halogen steht,
$R^4$ für Wasserstoff oder Alkyl steht,
$R^5$ für Wasserstoff, Alkyl oder gegebenenfalls substituiertes Aryl steht,
$R^6$ für Wasserstoff, Alkyl, Alkenyl, Alkinyl, Alkoxyalkyl, Alkylthioalkyl, Dialkylaminoalkyl, Halogenalkyl, Alkoxycarbonylalkyl, gegebenenfalls substituiertes Aryl oder gegebenenfalls substituiertes Aralkyl steht, und außerdem
$R^5$ und $R^6$ gemeinsam für eine Alkylen-Gruppe stehen, und
Z für Halogen steht,
dadurch gekennzeichnet, daß man

a) Anilinomethyloxim-ether der Formel

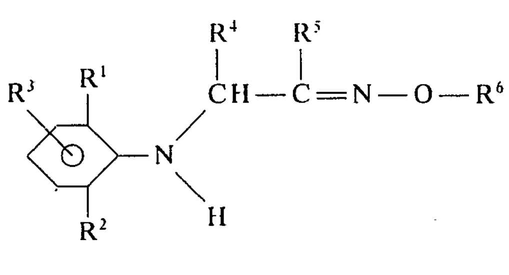

(II)

in welcher
$R^1$, $R^2$, $R^3$, $R^4$, $R^5$ und $R^6$ die oben angegebene Bedeutung haben,

mit Halogenessigsäurechloriden oder -bromiden bzw. -anhydriden der Formeln

$$Z—CH_2—CO—Cl(Br) \quad (IIIa)$$

bzw.

$$(Z—CH_2—CO)_2O \quad (IIIb)$$

in welchen
Z die oben angegebene Bedeutung hat,
in Gegenwart eines Verdünnungsmittels und gegebenenfalls in Gegenwart eines Säurebindemittels umsetzt, oder

b) Halogenacetanilide der Formel

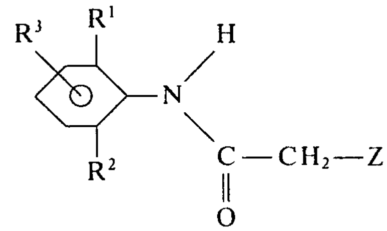

(IV)

in welcher
$R^1$, $R^2$, $R^3$ und Z die oben angegebene Bedeutung haben, mit substituierten Oxim-ethern der Formel

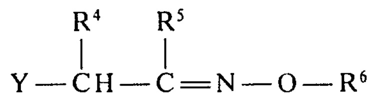

(V)

in welcher
$R^4$, $R^5$ und $R^6$ die oben angegebene Bedeutung haben, und
Y für Halogen, den Mesylat- oder Tosylat-Rest steht,
in Gegenwart eines Säurebinders und gegebenenfalls in Gegenwart eines Verdünnungsmittels umsetzt, oder

c) N-substituierte Halogenacetanilide der Formel

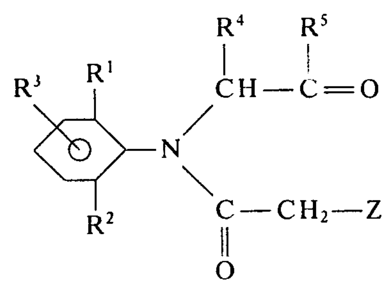

(VI)

in welcher $R^1$, $R^2$, $R^3$, $R^4$, $R^5$ und
Z die oben angegebene Bedeutung haben,
mit Salzen von Hydroxylamin-(Derivaten) der Formel

$$H_2N—O—R^6 \quad (VII)$$

in welcher
$R^6$ die oben angegebene Bedeutung hat,
in Gegenwart eines Verdünnungsmittels und in Gegenwart eines Säurebindemittels umsetzt, oder

d) Alkalisalze von Oximen der Formel

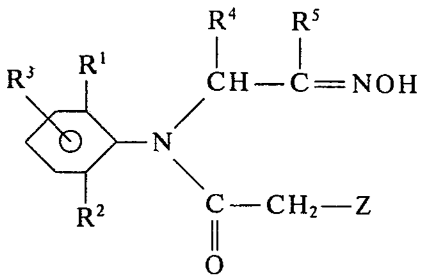

(VIII)

in welcher
$R^1$, $R^2$, $R^3$, $R^4$, $R^5$ und
Z die oben angegebene Bedeutung haben,
mit Halogeniden der Formel

$$X—R^7 \qquad (IX)$$

in welcher
$R^7$ für Alkyl, Alkenyl, Alkinyl, Alkoxyalkyl, Alkylthioalkyl, Dialkylaminoalkyl, Halogenalkyl, Alkoxycarbonylalkyl, gegebenenfalls substituiertes Aryl oder gegebenenfalls substituiertes Aralkyl steht und
X für Chlor oder Brom steht,
in Gegenwart eines organischen Verdünnungsmittels oder in Gegenwart eines organisch-anorganischen Zweiphasensystems in Gegenwart eines Phasentransferkatalysators umsetzt, wobei die Alkalisalze der Oxime der Formel (VIII) in situ erzeugt werden.

3. Herbizide Mittel, gekennzeichnet durch einen Gehalt an mindestens einem Oximether der Formel (I) gemäß Anspruch 1.

4. Verfahren zur Bekämpfung von Unkräutern, dadurch gekennzeichnet, daß man Oximether der Formel (I) gemäß Anspruch 1 auf Unkräuter und/oder deren Lebensraum einwirken läßt.

5. Verwendung von Oximethern der Formel (I) gemäß Anspruch 1 zur Bekämpfung von Unkräutern.

6. Verfahren zur Herstellung von herbiziden Mitteln, dadurch gekennzeichnet, daß man Oximether der Formel (I) gemäß Anspruch 1 mit Streckmitteln und/oder oberflächenaktiven Stoffen vermischt.

**Claims**

1. Oxime ethers of the formula

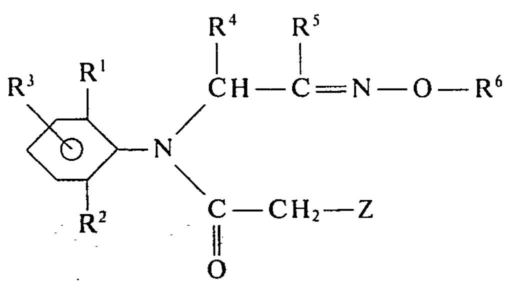

(I)

in which
$R^1$ represents hydrogen, alkyl or alkoxy,
$R^2$ represents hydrogen, alkyl, alkoxy or halogen,
$R^3$ represents hydrogen, alkyl, alkoxy or halogen,
$R^4$ represents hydrogen or alkyl,
$R^5$ represents hydrogen, alkyl or optionally substituted aryl,
$R^6$ represents hydrogen, alkyl, alkenyl, alkinyl, alkoxyalkyl, alkylthioalkyl, dialkylaminoalkyl, halogenoalkyl, alkoxycarbonylalkyl, optionally substituted aryl or optionally substituted aralkyl, and in addition
$R^5$ and $R^6$ together represent an alkylene group, and
Z represents halogen.

2. Process for the preparation of oxime ethers of the formula

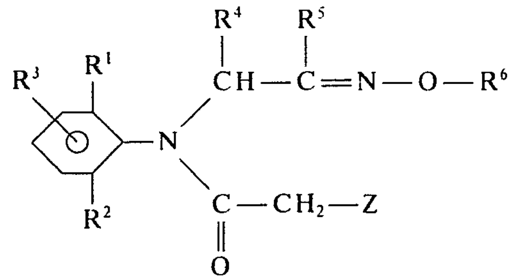

(I)

in which
$R^1$ represents hydrogen, alkyl or alkoxy,
$R^2$ represents hydrogen, alkyl, alkoxy or halogen,
$R^3$ represents hydrogen, alkyl, alkoxy or halogen,
$R^4$ represents hydrogen or alkyl,
$R^5$ represents hydrogen, alkyl or optionally substituted aryl,
$R^6$ represents hydrogen, alkyl, alkenyl, alkinyl, alkoxyalkyl, alkylthioalkyl, dialkylaminoalkyl, halogenoalkyl, alkoxycarbonylalkyl, optionally substituted aryl or optionally substituted aralkyl, and in addition
$R^5$ and $R^6$ together represent an alkylene group, and
Z represents halogen,
characterised in that
a) anilinomethyl oxime ethers of the formula

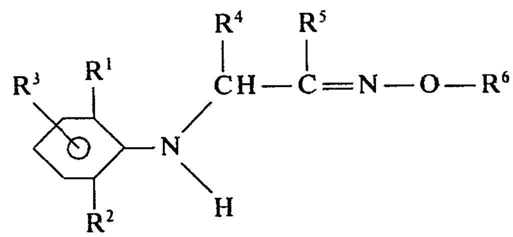

(II)

in which
$R^1$, $R^2$, $R^3$, $R^4$, $R^5$ and $R^6$ have the meaning indicated above,
are reacted with halogenoacetic acid chlorides or bromides or anhydrides of the formulae

$$Z—CH_2—CO—Cl(Br) \quad (IIIa)$$

or

$$(Z—CH_2—CO)_2O \quad (IIIb)$$

in which
Z has the meaning indicated above,
in the presence of a diluent and if appropriate in the presence of an acid-binding agent, or
b) halogenoacetanilides of the formula

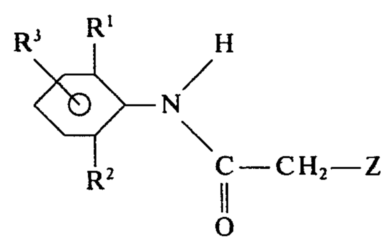

(IV)

in which
$R^1$, $R^2$, $R^3$ and Z have the meaning indicated above, are reacted with substituted oxime ethers of the formula

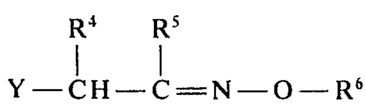

(V)

in which
$R^4$, $R^5$ and $R^6$ have the meaning indicated above and
Y represents halogen or the mesylate or tosylate radical,
in the presence of an acid-binding agent and if appropriate in the presence of a diluent, or

c) N-substituted halogenoacetanilides of the formula

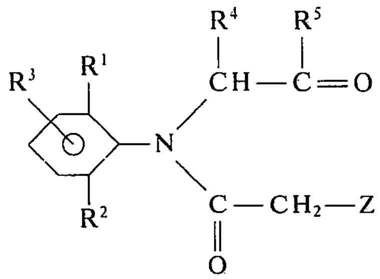

(VI)

in which
$R^1$, $R^2$, $R^3$, $R^4$, $R^5$ and Z have the meaning indicated above,
are reacted with salts of hydroxylamine (derivatives) of the formula

$$H_2N—O—R^6 \qquad (VII)$$

in which
$R^6$ has the meaning indicated above,
in the presence of a diluent and in the presence of an acidbinding agent, or

d) alkali metal salts of oximes of the formula

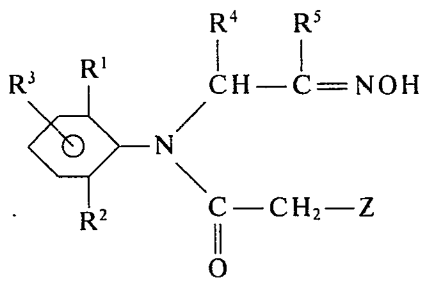

(VIII)

in which
$R^1$, $R^2$, $R^3$, $R^4$, $R^5$ and Z have the meaning indicated above,
are reacted with halides of the formula

$$X—R^7 \qquad (IX)$$

in which
$R^7$ represents alkyl, alkenyl, alkinyl, alkoxyalkyl, alkylthioalkyl, dialkylaminoalkyl, halogenoalkyl, alkoxycarbonylalkyl, optionally substituted aryl or optionally substituted aralkyl and
X represents chlorine or bromine,
in the presence of an organic diluent or in the presence of an organic-inorganic two-phase system in the presence of a phase transfer catalyst, the alkali metal salts of the oximes of the formula (VIII) being produced in situ.

3. Herbicidal agents, characterised in that they contain at least one oxime ether of the formula (I) according to Claim 1.

4. Process for combating weeds, characterised in that oxime ethers of the formula (I) according to Claim 1 are allowed to act on weeds and/or their environment.

5. Use of oxime ethers of the formula (I) according to Claim 1 for combating weeds.

6. Process for the preparation of herbicidal agents, characterised in that oxime ethers of the formula (I) according to Claim 1 are mixed with extenders and/or surface-active substances.

**Revendications**

1. Oxime-éthers de formule

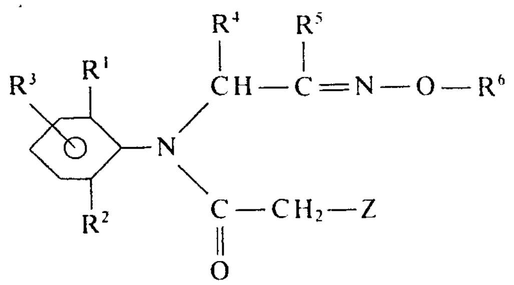

(I)

dans laquelle
$R^1$ représente de l'hydrogène, un alcoyle ou alcoxy,
$R^2$ représente de l'hydrogène, un alcoyle, alcoxy ou de l'halogène,
$R^3$ représente de l'hydrogène, un alcoyle, alcoxy ou de l'halogène,
$R^4$ représente de l'hydrogène ou un alcoyle,
$R^5$ représente de l'hydrogène, un alcoyle ou un aryle éventuellement substitué,
$R^6$ représente de l'hydrogène, un alcoyle, alcényle, alcinyle, alcoxyalcoyle, alcoylthioalcoyle, dialcoylaminoalcoyle, halogénoalcoyle, alcoxycarbonylalcoyle, un aryle éventuellement substitué ou un aralcoyle éventuellement substitué et, en outre,
$R^5$ et $R^6$ représentent ensemble un groupe alcoylène et
Z représente de l'halogène,

2. Procédé de préparation d'oxime-éthers de formule

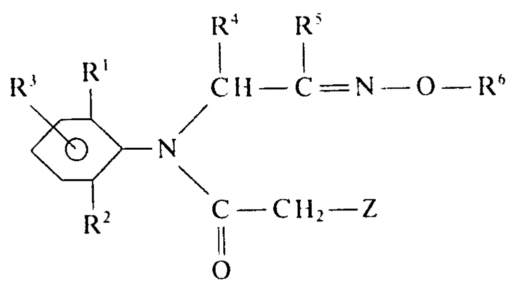

(I)

dans laquelle
$R^1$ représente de l'hydrogène, un alcoyle ou un alcoxy,
$R^2$ représente de l'hydrogène, un alcoyle, alcoxy ou de l'halogène,
$R^3$ représente de l'hydrogène, un alcoyle, alcoxy ou de l'halogène,
$R^4$ représente de l'hydrogène ou un alcoyle,
$R^5$ représente de l'hydrogène, un alcoyle ou aryle éventuellement substitué,
$R^6$ représente de l'hydrogène, un alcoyle, alcényle, alcinyle, alcoxyalcoyle, alcoylthioalcoyle, dialcoylaminoalcoyle, halogénoalcoyle, alcoxycarbonylalcoyle, aryle éventuellement substitué ou aralcoyle éventuellement substitué et, en outre,
$R^5$ et $R^6$ représentent ensemble un groupe alcoylène et
Z représente de l'halogène,
caractérisé en ce que
a) on fait réagir des anilinométhyloxime-éthers de formule:

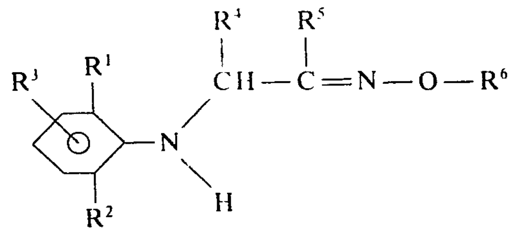

(II)

dans laquelle
$R^1$, $R^2$, $R^3$, $R^4$, $R^5$ et $R^6$ ont la signification indiquée plus haut,
avec des chlorures ou bromures ou anhydrides d'acides halogénoacétiques de formules:

$$Z-CH_2-CO-Cl(Br) \qquad \text{(IIIa)}$$

ou

$$(Z-CH_2-CO)_2O \qquad \text{(IIIb)}$$

dans lesquelles
Z a la signification indiquée plus haut
en présence d'un diluant et éventuellement en présence d'un agent fixateur d'acide, ou

b) on fait réagir des halogénoacétanilides de formule:

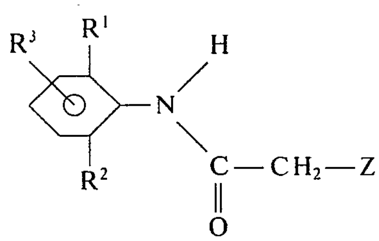

(IV)

dans laquelle
$R^1$, $R^2$, $R^3$ et Z ont la signification indiquée plus haut, avec des oxime-éthers substitués de formule:

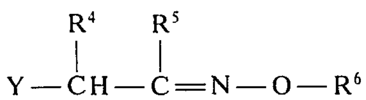

(V)

dans laquelle
$R^4$, $R^5$ et $R^6$ ont la signification indiquée plus haut et
Y représente de l'halogène, le radical mésylate ou tosylate,
en présence d'un fixateur d'acide et éventuellement en présence d'un diluant, ou

c) on fait réagir des halogénoacétanilides N-substituées de formule:

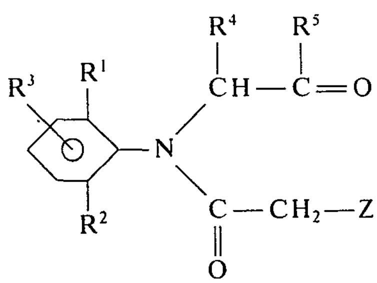

(VI)

dans laquelle
$R^1$, $R^2$, $R^3$, $R^4$, $R^5$ et Z ont la signification indiquée plus haut,
avec des sels de (dérivés) d'hydroxylamines de formule:

$$H_2N-O-R^6 \qquad \text{(VII)}$$

dans laquelle
$R^6$ a la signification indiquée plus haut,
en présence d'un diluant et en présence d'un agent fixateur d'acide, ou

d) on fait réagir des sels alcalins d'oximes de formule:

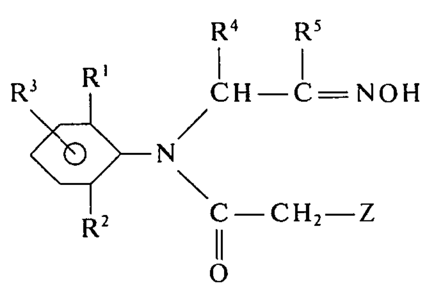

(VIII)

dans laquelle
$R^1$, $R^2$, $R^3$, $R^4$, $R^5$ et Z ont la signification indiquée plus haut,
avec des halogénures de formule:

$$X — R^7 \qquad (IX)$$

dans laquelle
$R^7$ représente un alcoyle, alcényle, alcinyle, alcoxyalcoyle, alcoylthioalcoyle, dialcoylaminoalcoyle, halogénoalcoyle, alcoxycarbonylalcoyle, aryle éventuellement substitué ou aralcoyle éventuellement substitué et
X représente du chlore ou du brome,
en présence d'un diluant organique ou en présence d'un système organo-mineral à deux phases et en présence d'un catalyseur de transfert de phase, les sels alcalins des oximes de formule (VIII) étant engendrés in situ.

3. Agents herbicides, caractérisés par une teneur en au moins un oxime-éther de formule (I) selon la revendication 1.

4. Procédé pour combattre les mauvaises herbes, caractérisé en ce qu'on fait agir un oxime-éther de formule (I) selon la revendication 1 sur des mauvaises herbes et/ou sur leur espace vital.

5. Utilisation des oxime-éthers de formule (I) selon la revendication 1 pour combattre les mauvaises herbes.

6. Procédé de fabrication d'agents herbicides, caractérisé en ce qu'on mélange des oximeéthers de formule (I) selon la revendication 1 avec des diluants et/ou des substances tensioactives.